# EUROPÄISCHE PATENTSCHRIFT

(11) **EP 3 983 799 B1**
(45) Veröffentlichungstag und Bekanntmachung des Hinweises auf die Patenterteilung: **07.10.2026**
(21) Anmeldenummer: 20753272.2
(22) Anmeldetag: 15.06.2020
(51) Int. Cl.: G01N 33/204, G01N 23/20, G01N 23/02, G01N 27/72, G01N 21/17

(54) **VORRICHTUNG UND VERFAHREN ZUM BERÜHRUNGSLOSEN ERMITTELN VON ZUMINDEST EINER EIGENSCHAFT EINES METALLPRODUKTS**
DEVICE AND METHOD FOR THE CONTACTLESS DETERMINATION OF AT LEAST ONE PROPERTY OF A METAL PRODUCT
DISPOSITIF ET PROCÉDÉ POUR DÉTERMINER SANS CONTACT AU MOINS UNE PROPRIÉTÉ D'UN PRODUIT MÉTALLIQUE

(30) Priorität: 14.06.2019 DE 102019208705
(43) Veröffentlichungstag der Anmeldung: 20.04.2022
(73) Patentinhaber: SMS group GmbH, 41069 Mönchengladbach (DE); Drever International S.A., 4031 Liège (Angleur) (BE)
(72) Erfinder: LHOEST, Alexandre, 4700 Eupen (BE); PENSIS, Olivier, 4420 Montegnee (BE); SOMMERS, Ulrich, 40627 Düsseldorf (DE); HOUSEN, Vincent, 4450 Juprelle (BE)
(74) Vertreter: Gevers Patents
(86) Internationale Anmeldenummer: PCT/EP2020/066494
(87) Internationale Veröffentlichungsnummer: WO 2020/249820

(56) Entgegenhaltungen:
- EP-A2- 0 941 806
- EP-B1- 0 941 806
- WO-A1-2017/202904
- WO-A1-2019/003727
- WO-A2-01/46682
- JP-A- S5 662 917
- US-A- 5 654 977
- D WEHRHAHN: "Berührungslose Lasermessung in der Aluminium-und Stahlindustrie", 15 April 2019 (2019-04-15), XP055757417, Retrieved from the Internet <URL:https://www.drwehrhahn.de/fileadmin/redakteure/dokumente/de/anwendungsbereiche/stahl_de.pdf> [retrieved on 20201207]

## Beschreibung

Die Erfindung betrifft eine Vorrichtung zum berührungslosen Ermitteln von zumindest einer Eigenschaft eines Metallprodukts während einer metallurgischen Herstellung des Metallprodukts nach dem Oberbegriff von Anspruch 1, und ein entsprechendes Verfahren nach dem Oberbegriff von Anspruch 15.

Nach dem Stand der Technik ist es bei der Herstellung von metallischen Produkten bekannt, dass beispielsweise zum Zwecke einer Qualitätssicherung auch Eigenschaften der Metallprodukte ermittelt bzw. überprüft werden. Zu diesem Zweck werden Messvorrichtungen eingesetzt, die eine berührungslose und zerstörungsfreie Bestimmung von Eigenschaften des Metallprodukts gewährleisten. Im Zusammenhang mit Ofeneinrichtungen oder dergleichen, in denen zumeist eine hohe Temperatur herrscht, sind solche Messvorrichtungen in der Regel außerhalb des Gehäuses des Ofens angeordnet. Hierbei stellt sich die Problematik, dass die Komponenten der Messvorrichtung nicht ohne Weiteres in direkter Nähe des Metallprodukts angeordnet werden können, wobei des Weiteren eine aufwendige Kühlung für die Komponenten der Messvorrichtung mit einem entsprechenden Temperaturmanagement erforderlich ist, damit diese Komponenten durch die hohe Temperatur eines Ofens bzw. des Metallprodukts keinen Schaden nehmen.

Aus WO 2019/228692 A1 ist es bekannt, den Austenitgehalt eines Metallprodukts durch Einsatz von elektromagnetischer Strahlung in Echtzeit zu bestimmen. Zu diesem Zweck kommen zwei mit Wechselspannung gespeiste Spulen zum Einsatz, zwischen denen das Metallprodukt hindurch bewegt wird. Eine der beiden Spulen sendet eine elektromagnetische Strahlung auf das Metallprodukt aus, wobei das resultierende Wellenmuster der elektromagnetischen Strahlung, die durch das Metallprodukt hindurchgetreten ist, von der jeweils anderen Spule empfangen wird.

Der Abstand zwischen den beiden Spulen ist auf einen bestimmten Wert eingestellt, wobei eine Veränderung dieses Abstands - einmal eingestellt - nicht vorgesehen ist. Nach dem Stand der Technik kann das berührungslose und zerstörungsfreie Bestimmen der Eigenschaften eines Metallprodukts während dessen metallurgischer Herstellung auch nach dem Prinzip der Röntgenstrahlbeugung durchgeführt werden. Dies ist z.B. aus WO 2017/202904 A1 bekannt, bei der die Mikrostruktur eines Metallprodukts durch Einsatz einer Röntgenquelle und eines Röntgendetektors ermittelt wird, wobei die Röntgenquelle und der Röntgendetektor jeweils in einer aktiv gekühlten Aufnahmekammer angeordnet sind. Das zu überprüfende Metallprodukt wird zwischen der Röntgenquelle und dem Röntgendetektor vorbeibewegt, wobei ein Nachteil darin besteht, dass ein Abstand zwischen der Röntgenquelle und dem Röntgendetektor relativ zueinander bzw. der Abstand dieser beiden Messkomponenten zum Metallprodukt unveränderlich ist. Bei der Technologie gemäß WO 2017/202904 A1 stellt sich nachteilig das Problem, dass ein Abstand zwischen einerseits der Röntgenquelle und dem Röntgendetektor und andererseits dem Metallprodukt vergleichsweise groß ist und anlagentechnisch nicht auf kleinere Werte gebracht werden kann.

Aus JP S 56 62917 A ist ebenfalls die Bestimmung von Werkstoffeigenschaften eines Metallprodukts nach dem Prinzip der Röntgenstrahlbeugung bekannt. Hierbei ist eine Ofeneinrichtung, durch die ein Metallprodukt für ein Anlassen bzw. "Quenching" hindurchgeführt wird, mit einer Messvorrichtung ausgestattet, mittels der Röntgenstrahlen auf das Metallprodukt gerichtet werden, um dadurch konkret den Austenitgehalt des Metallprodukts zu bestimmen. Diese Messvorrichtung ist außerhalb des Gehäuses der Ofeneinrichtung und hierbei in einer Einschnürung bzw. Engstelle des Gehäuses fest angeordnet, so dass dadurch ein Abstand der Messvorrichtung zum Metallprodukt verringert ist. Durch die Geometrie der besagten Engstelle des Gehäuses der Ofeneinrichtung ist ein Abstand der Messvorrichtung zum Metallprodukt fest vorgegeben und kann nicht verändert werden.

Das Dokument WO 01/46682 A2 beschreibt ein Verfahren zur in situ-Ermittlung des Umwandlungsgrads einer nichtmagnetischen Phase in eine ferromagnetische Phase eines metallischen Werkstücks, mit einem Wirbelstrommessverfahren, bei welchem mit zwei Abstandsmessspulen, die unterschiedlich weit von dem Werkstück beabstandet sind, abstandsabhängige Signale ermittelt werden, anhand welcher ein abstandsabhängiger Rechenwert ermittelt wird, anhand welchem zusammen mit einem weiteren Messsignal einer Detektormessspule, die das aus einer Beaufschlagung des Werkstücks mit einem Erregerfeld resultierende Antwortfeld misst, ein Masswert für den Umwandlungsgrad bestimmt wird.

Das Dokument JPS5662917A bezieht sich auf die Verbesserung der Festigkeit und Duktilität durch die Steuerung der Abschreckgeschwindigkeit und die richtige Einstellung der Martensitbildungsmenge zum Zeitpunkt des kontinuierlichen Glühens eines Stahlblechs durch Abschrecken desselben aus dem Temperaturbereich von zwei Phasen, Ferrit und Austenit. Es wird offenbart: Zum Zeitpunkt des kontinuierlichen Glühens eines Stahlblechs 8 wird es auf den Temperaturbereich erwärmt, in dem die beiden Phasen Ferrit und Austenit in der Struktur in einer Heizzone koexistieren, wodurch es in eine zweiphasige Struktur gebracht wird, woraufhin es in der Abschreckzone 28 im Anschluss an die Durchwärmungszone abgeschreckt wird. In diesem Fall wird die Austenitmenge in der Stahlplatte, die eine Kühleinheit 50 passiert hat, mit einer Austenitmengenmesseinrichtung 56 durch ein Röntgenbeugungsverfahren gemessen, die in der Abschreckzone 28 vorgesehen ist. Dieser Messwert wird zusammen mit dem von einem Thermometer 54 gemessenen Temperaturwert des Stahlblechs in eine Recheneinheit 58 eingegeben. Ein oberstromseitiger Kühler 50 wird von diesem Rechner 59 so gesteuert, dass die Temperatur des Stahlblechs die Temperatur knapp über dem Beginn der Martensitabscheidung erreicht; außerdem wird die Stahlblech-Fördergeschwindigkeit so gesteuert, dass die mit der Messvorrichtung 56 ermittelte Austenitmenge die vorbestimmte Menge wird, die der Martensitmenge im Produkt entspricht, wodurch die Stahlblech-Kühlrate richtig gesteuert wird.

Das Dokument "D Wehrhahn: ,Berührungslose Lasermessung in der Aluminium- und Stahlindustrie', XP055757417" beschreibt den Einsatz der berührungslosen Lasermessung in der Aluminium- und Stahlindustrie. In diesem Dokument werden Sensoranordnungen für die Dickenmessung beschrieben. Bei der Dickenmessung mit zwei Sensoren wird das Produkt zwischen zwei Sensoren hindurchgeführt, wobei sich einer über und einer unter dem Material befindet. Die Berechnung der beiden Sensorwerte ergibt die Dicke. Für die Messung von warmen und heißen Materialien stehen luft- und wassergekühlte Sensoren zur Verfügung.

Das Dokument EP0941806A2 beschreibt eine Wafer-Poliervorrichtung mit beweglichem Fenster, die zur In-situ-Überwachung eines Wafers während der CMP-Bearbeitung verwendet werden kann. Während des größten Teils des CMP-Vorgangs bleibt das Fenster unter einer Polierfläche einer Poliervorrichtung, um das Fenster vor den schädlichen Auswirkungen des Poliervorgangs zu schützen. Wenn sich das Fenster in Position zwischen dem Wafer und einem Messsensor bewegt, wird das Fenster näher an die Polierfläche herangeführt. In dieser Position wird zumindest ein Teil des in der Vertiefung über dem Fenster angesammelten Poliermittels entfernt und es kann eine In-situ-Messung mit geringerer Beeinträchtigung durch das Poliermittel durchgeführt werden. Nachdem das Fenster vom Wafer und vom Messsensor entfernt wurde, bewegt es sich weiter vom Wafer und von der Polierfläche weg. Mit einem solchen beweglichen Fenster werden die Einschränkungen aktueller Poliergeräte überwunden.

Entsprechend liegt der Erfindung die Aufgabe zugrunde, eine Technologie zum berührungslosen und störungsfreien Bestimmen der Werkstoffeigenschaften eines Metallprodukts zu schaffen, mit der das Ermitteln von zumindest einer Eigenschaft des Metallprodukts im Vergleich zum vorstehend genannten Stand der Technik optimiert und mit größerer Variabilität möglich ist.

Diese Aufgabe wird durch eine Vorrichtung mit den Merkmalen von Anspruch 1 und durch ein Verfahren mit den Merkmalen von Anspruch 15 gelöst. Vorteilhafte Weiterbildungen der Erfindung sind in den abhängigen Ansprüchen definiert.

Die Erfindung sieht eine Vorrichtung zum berührungslosen Ermitteln von zumindest einer Eigenschaft eines Metallprodukts während einer metallurgischen Herstellung des Metallprodukts vor. Diese Vorrichtung umfasst ein Gehäuse, durch welches das Metallprodukt hindurch bewegt werden kann, und zumindest eine Messvorrichtung bestehend aus einer Sendeeinheit und einer Empfangseinheit, wobei zumindest von der Sendeeinheit ein elektromagnetisches Feld erzeugt und auf das Metallprodukt gerichtet wird und dadurch in dem Material des Metallprodukts eine physikalische Wechselwirkung induziert wird. Ein verbleibender und/oder resultierender Teil dieser physikalischen Wechselwirkung kann von der Empfangseinheit empfangen werden. In der Wandung des Gehäuses sind eine erste Öffnung und eine zweite Öffnung ausgebildet. Hierbei ist die besagte Sendeeinheit der Messvorrichtung der ersten Öffnung zugeordnet, so dass das von der Sendeeinheit erzeugte elektromagnetische Feld und/oder dessen zugehörige Feldlinien auf der Seite der ersten Öffnung auf das Metallprodukt auftreffen. Des Weiteren ist die Empfangseinheit der Messvorrichtung der zweiten Öffnung zugeordnet, so dass der verbleibende und/oder resultierende Teil der in dem Material des Metallprodukts induzierten physikalischen Wechselwirkung von der Empfangseinheit auf der Seite der zweiten Öffnung empfangen bzw. detektiert werden kann. Die erfindungsgemäße Vorrichtung umfasst zumindest eine insbesondere außerhalb des Gehäuses vorgesehene Verstelleinrichtung, mit der zumindest eine Komponente der Messvorrichtung bestehend aus der Sendeeinheit und/oder der Empfangseinheit im Bereich einer Öffnung des Gehäuses oder angrenzend hierzu und relativ zu der Wandung des Gehäuses bzw. relativ zu dem innerhalb des Gehäuses geführten Metallprodukts bewegt werden kann, um dadurch für diese Komponente, d.h. für die Sendeeinheit und/oder die Empfangseinheit einen vorbestimmten Abstand zu dem innerhalb des Gehäuses bewegten Metallprodukt einzustellen oder gezielt zu verändern.

In gleicher Weise sieht die Erfindung auch ein Verfahren zum berührungslosen Ermitteln von zumindest einer Eigenschaft eines Metallprodukts während einer metallurgischen Herstellung des Metallprodukts vor. Bei diesem Verfahren wird das Metallprodukt durch ein Gehäuse einer Vorrichtung bewegt, wobei es sich bei dieser Vorrichtung um eine solche nach einem der Ansprüche 1 bis 14 handeln kann. Jedenfalls wird bei dem erfindungsgemäßen Verfahren zumindest eine Messvorrichtung bestehend aus einer Sendeeinheit und einer Empfangseinheit eingesetzt, wobei zumindest von der Sendeeinheit ein elektromagnetisches Feld erzeugt und auf das Metallprodukt gerichtet wird, wodurch in dem Material des Metallprodukts eine physikalische Wechselwirkung induziert bzw. hervorgerufen wird. Anschließend wird der verbleibende und/oder resultierende Teil dieser physikalischen Wechselwirkung von der Empfangseinheit empfangen. Das erfindungsgemäße Verfahren zeichnet sich dadurch aus, dass zumindest eine Komponente der Messvorrichtung bestehend aus der Sendeeinheit und/oder der Empfangseinheit relativ zum Gehäuse bzw. des darin bewegten Metallprodukts und im Bereich einer Öffnung des Gehäuses oder angrenzend hierzu bewegt wird, um dadurch für diese Komponente einen vorbestimmten Abstand zum Metallprodukt einzustellen oder gezielt zu verändern.

In Bezug auf das Merkmal "metallurgische Herstellung eines Metallprodukts" darf an dieser Stelle darauf hingewiesen werden, dass hierzu im Sinne der vorliegenden Erfindung auch eine Wärmebehandlung des metallischen Produkts gehört, die in bzw. mit einer Ofeneinrichtung durchgeführt wird und beispielsweise ein Anlassen bzw. Glühen des Metallprodukts bezweckt. Entsprechend kann das Gehäuse, durch welches hindurch das Metallprodukt bewegt bzw. geführt wird, Teil eines Ofens zum Wärmebehandeln sein.

Ergänzend oder alternativ kann nach der vorliegenden Erfindung auch vorgesehen sein, dass das Gehäuse, durch welches hindurch das Metallprodukt bewegt bzw. geführt wird, Teil einer Anlage zum Beschichten des Metallprodukts ist.

Des Weiteren darf an dieser Stelle gesondert darauf hingewiesen werden, dass das Metallprodukt, dessen Eigenschaften mit der erfindungsgemäßen Vorrichtung ermittelt werden können, nicht als solches Teil dieser Vorrichtung ist. Vielmehr ist das Gehäuse der Vorrichtung dazu geeignet bzw. ausgelegt, dass das Metallprodukt während seiner metallurgischen Herstellung in einer Bewegungsrichtung durch das Gehäuse hindurchbewegt bzw. geführt wird.

Im Hinblick auf die vorstehend genannte Wärmebehandlung des metallischen Produkts ist somit für die Vorrichtung und das entsprechende Verfahren gemäß der vorliegenden Erfindung zu verstehen, dass das berührungslose Ermitteln von zumindest einer Eigenschaft des Metallprodukts insbesondere auch dann durchgeführt werden kann, wenn das Metallprodukt entweder vergleichsweise hohen Temperaturen ausgesetzt ist, beispielsweise in einem Ofen zur Wärmebehandlung, und/oder selber noch eine vergleichweise hohe Temperatur aufweist.

Der Erfindung liegt die wesentliche Erkenntnis zugrunde, dass es möglich ist, zumindest eine Komponente der Messvorrichtung, d.h. die Sendeeinheit oder die Empfangseinheit, oder aber beide dieser Komponenten der Messvorrichtung, d.h. die Sendeeinheit und die Empfangseinheit, relativ zur Wandung des Gehäuses, durch welches das Metallprodukt hindurch geführt wird, zu bewegen bzw. zu verstellen. Durch eine solche Bewegung kann der Abstand von Sendeeinheit und Empfangseinheit relativ zueinander, und/oder damit gleichzeitig auch ein Abstand der Sendeeinheit und/oder der Empfangseinheit zu dem innerhalb des Gehäuses geführten Metallprodukts, gezielt eingestellt bzw. geändert werden, beispielsweise auch "Online" bzw. während einer laufenden Messung der Werkstoffeigenschaften des Metallprodukts und/oder während dessen metallurgischer Herstellung.

In Bezug auf einen vorbestimmten Abstand zwischen einerseits den Komponenten der Messvorrichtung, d.h. der Sendeeinheit und/oder der Empfangseinheit, und andererseits dem Metallprodukt darf an dieser Stelle darauf hingewiesen werden, dass dieser Abstand beispielsweise 100 mm betragen kann. Mittels der vorliegenden Erfindung kann erreicht werden, dass dieser Abstand möglichst klein ist und einen Wert annehmen kann, der vorzugsweise kleiner als 50 mm, weiter vorzugsweise kleiner als 30 mm, weiter vorzugsweise kleiner als 20 mm ist und weiter vorzugsweise etwa 10 mm betragen kann. In diesem Zusammenhang wird auch darauf hingewiesen, dass der vorbestimmte Abstand auch an das Design bzw. die Abmessungen von Sendeeinheit und/oder Empfangseinheit angepasst ist. Beispielsweise ist der Abstand der Empfangseinheit zum Metallprodukt auch von den Abmessungen bzw. der Größe der Empfangseinheit abhängig. Dies gilt in gleicher Weise auch für die Sendeeinheit. Jedenfalls wird der vorbestimmte Abstand zwischen einerseits der Sendeeinheit und/oder Empfangseinheit und andererseits dem Metallprodukt derart eingestellt, dass damit auch die jeweiligen Vorgaben in Bezug auf die Produktstabilität berücksichtigt werden.

In vorteilhafter Weiterbildung der Erfindung können die Verstelleinrichtungen für ein Bewegen der Komponenten der Messvorrichtung jeweils eine hohe Ansprechgeschwindigkeit und eine große Stellgeschwindigkeit aufweisen. Unter Berücksichtigung dessen kann ein vorbestimmter Abstand zwischen einerseits der Sendeeinheit und/oder der Empfangseinheit und andererseits dem Metallprodukt auch auf Werte von weniger als 10 mm eingestellt werden. Einzelheiten hierzu sind nachfolgend noch gesondert erläutert.

Bei der Erfindung weist das Gehäuse im Bereich der ersten Öffnung und/oder der zweiten Öffnung eine Engstelle auf. Indem eine Komponente der Messvorrichtung, d.h. die Sendeeinheit und/oder die Empfangseinheit, im Bereich oder innerhalb dieser Öffnungen des Gehäuses oder angrenzend hierzu in Richtung des Metallprodukts bewegt wird, was dann in gleicher Weise auch auf die Engstelle zutrifft, lässt sich dank der besagten Engstelle ein resultierender Abstand zwischen einerseits der Sendeeinheit und/oder der Empfangseinheit und andererseits dem Metallprodukt weiter vermindern.

In Bezug auf das Merkmal "vorbestimmter Abstand" zwischen einer Komponente der Messvorrichtung (= Sendeeinheit und/oder Empfangseinheit) und dem Metallprodukt darf an dieser Stelle gesondert darauf hingewiesen werden, dass dieser Abstand im Sinne der vorliegenden Erfindung jeweils in Anpassung an das physikalische Prinzip gewählt ist, mit dem von der Sendeeinheit beispielsweise Wellen erzeugt und auf das Metallprodukt gerichtet werden. Anders ausgedrückt, ist dieser vorbestimmte Abstand zwischen einerseits der Sendeeinheit/Empfangseinheit und andererseits dem Metallprodukt jeweils an den Typ des von der Sendeeinheit erzeugten elektromagnetischen Felds (z.B. Röntgenstrahlung oder Laserstrahlung) oder an das durch die Sendeeinheit erzeugte Magnetfeld, welches auf das Metallprodukt wirkt, angepasst. In der Regel ist dieser vorbestimmte Abstand möglichst klein gewählt und kann einen der vorstehend genannten beispielhaften Werte annehmen.

Das Bewegen der Sendeeinheit und/oder der Empfangseinheit relativ zur Wandung des Gehäuses erfolgt vorzugsweise translatorisch, beispielsweise unter Verwendung von zumindest einer Verstelleinrichtung, die insbesondere außerhalb des Gehäuses, durch welches hindurch das Metallprodukt bewegt bzw. geführt wird, angeordnet ist und mit der Sendeeinheit bzw. Empfangseinheit geeignet in Wirkverbindung steht. Eine solche Verstelleinrichtung kann teleskopartig ausgebildet sein, wodurch mit einfachen Mitteln für die damit verbundene Sendeeinheit bzw. Empfangseinheit ein großer Verstellweg für ein Bewegen hinein in das Gehäuse oder heraus aus dem Gehäuse erreicht wird. Eine solche teleskopartige Verstelleinrichtung hat zudem den Vorteil, dass sie lediglich einen geringen Bauraum beansprucht.

Ein weiterer Vorteil in Bezug auf die vorstehend genannte Beweglichkeit der Komponenten der Messvorrichtung besteht darin, dass es damit möglich ist, die Sendeeinheit und/oder die Empfangseinheit, vorzugsweise beide dieser Einheiten zusammen, aus dem Gehäuse der Vorrichtung heraus zu bringen und anschließend von dem Gehäuse zu beabstanden und/oder zu entfernen. Hierdurch vereinfachen sich dann mögliche Kalibrierungsarbeiten und/oder Wartungsarbeiten für diese Komponenten der Messvorrichtung.

Das Prinzip eines berührungslosen Ermittelns von zumindest einer Eigenschaft des Metallprodukts während seiner metallurgischen Herstellung beruht darauf, dass von der Sendeeinheit ein elektromagnetisches Feld erzeugt wird, das auf das Metallprodukt gerichtet wird. Anders ausgedrückt, ist bzw. wird das Metallprodukt in Bezug auf die Sendeeinheit derart angeordnet bzw. bewegt, dass sich das Metallprodukt im Einflussbereich der Feldlinien des von der Sendeeinheit erzeugten elektromagnetischen Felds befindet. Auf Grundlage dessen wird dann in dem Material des Metallprodukts die genannte physikalische Wechselwirkung hervorgerufen bzw. induziert.

Die vorstehend genannte physikalische Wechselwirkung kann gemäß der vorliegenden Erfindung auf folgenden technischen Aspekten beruhen:
- Durchlassen einer elektromagnetischen Strahlung, insbesondere Röntgenstrahlung, durch das Material des Metallprodukts hindurch ("Transmisions- bzw. Durchlassprinzip"), wobei der Teil der Strahlung (vorzugsweise Röntgenstrahlung), der durch das Metallprodukt hindurchtritt, von den Eigenschaften des Metallprodukts beeinflusst wird;
- Reflektieren einer elektromagnetischen Strahlung, insbesondere Laserstrahlung, an dem Material des Metallprodukts und/oder an dessen Oberfläche ("Reflexionsprinzip"), wobei der reflektierte Teil der Strahlung (vorzugsweise Laserstrahlung) von den Eigenschaften des Metallprodukts beeinflusst wird;
- Magnetisieren des Metallprodukts, das entsprechend aus einem magnetisierbaren Metall wie insbesondere Stahl besteht, durch das von der Sendeeinheit erzeugten elektromagnetischen Feld, wobei die magnetische Restfeldstärke und/oder deren Gradient, die bzw. der von der Empfangseinheit detektiert werden kann, von den Eigenschaften des Metallprodukts beeinflusst wird;
- Einbringen von elektromagnetischer Strahlung insbesondere in Form von Laserstrahlung, die durch das von der Sendeeinheit erzeugte elektromagnetische Feld ausgesendet wird, in das Material des Metallprodukts, wobei in Folge dessen in dem Material des Metallprodukts ein lokales Ultraschall-Feld erzeugt wird, das von der Empfangseinheit, beispielsweise ebenfalls auf Grundlage einer auf das Metallprodukt gerichteten Laserstrahlung, gemessen bzw. detektiert werden kann.

Grundsätzlich wird in Bezug auf die Strahlung, welche von dem von der Sendeeinheit erzeugten elektromagnetischen Feld ausgeht, angemerkt, dass es sich hierbei um jedwede Form einer elektromagnetischen Strahlung handeln kann, mit der das Ermitteln von zumindest einer Eigenschaft des Metallprodukts möglich ist, beispielsweise - wie vorstehend bereits genannt - Röntgenstrahlung oder Laserstrahlung, oder alternativ auch Mikrowellen, Infrarot oder mit Wellenlängen im sichtbaren Bereich.

In Bezug auf die elektromagnetische Strahlung, die von der Sendeeinheit wie vorstehend erläutert erzeugt werden können, versteht sich für den Fall, dass die Sendeeinheit und Empfangseinheit auf jeweils entgegengesetzten Seiten des Metallprodukts angeordnet sind, dass die von der Sendeeinheit erzeugten Wellen, vorzugsweise von Röntgenstrahlung, durch das Metallprodukt hindurchtreten, wobei dann das verbleibende und/oder resultierende Wellenmuster von der Empfangseinheit auf der entgegengesetzten Seite des Metallprodukts durch die Empfangseinheit empfangen wird.

Für den Fall, dass die Sendeeinheit und die Empfangseinheit jeweils auf der gleichen Seite des Metallprodukts angeordnet sind, wird die von der Sendeeinheit erzeugte Strahlung, vorzugsweise Laserstrahlung, an einer Oberfläche des Metallprodukts reflektiert, wobei anschließend das verbleibende und/ oder resultierende Wellenmuster beispielsweise der Laserstrahlung von der Empfangseinheit empfangen wird. Für diesen Fall kann auch vorgesehen sein, dass die erste und zweite Öffnung des Gehäuses zu einer gemeinsamen Öffnung zusammengefasst sind. Dies bedeutet, dass dann an der Seite des Gehäuses, an welcher die Sendeeinheit und die Empfangseinheit angeordnet sind, für diese Komponenten der Messvorrichtung eine gemeinsame und entsprechend ausreichend große Öffnung ausgebildet ist.

In vorteilhafter Weiterbildung der Erfindung können Sendeeinheit und Empfangseinheit Teil eines IMPOC-Messkopfes sein. Das IMPOC-Messprinzip (IMPOC = "Impulse-Magnetic-Process-Online-Controller") beruht darauf, dass beispielsweise ein Stahlband mit Hilfe von stromdurchflossenen Spulen in regelmäßigen Abständen stoßmagnetisiert und die magnetische Restfeldstärke der lokal erzeugten Magnetisierung bzw. ein hieraus berechneter Gradient mittels einer Empfängereinheit in Form eines Magnetfeldsensors gemessen wird. Konkret werden dem gemessenen Wert der magnetischen Restfeldstärke bzw. dem berechneten Gradienten über Korrelationsbeziehungen die mechanische Festigkeit des Abschnitts des untersuchten Metallprodukts zugeordnet, wobei diese mechanische Festigkeit insbesondere die Zugfestigkeit und die Streckgrenze des Materials des jeweiligen Metallprodukts umfasst. Anders ausgedrückt, werden die resultierenden bzw. verbleibenden magnetischen Eigenschaften des Metallprodukts durch die Empfängereinheit gemessen (Remanenz oder Hysteresekurve).

Beim IMPOC-Verfahren wird die Restfeldstärke in der Einheit [A/m²] gemessen.

Das IMPOC-Messprinzip ist auf magnetisierbare Stahlsorten beschränkt, wobei zugehörige Messeinrichtungen im Handel erhältlich sind.

In Bezug auf einen IMPOC-Messkopf darf ergänzend darauf hingewiesen werden, dass hierbei die Sendeeinheit in Form einer Magnetisierungsspule, und die Empfangseinheit in Form eines Magnetfeldsensors ausgebildet sind. Falls ein solcher IMPOC-Messkopf an einer Seite des Metallprodukts angeordnet ist, versteht sich im Hinblick darauf, dass wie erläutert die Magnetisierungsspule und der Magnetfeldsensor in diesen Messkopf integriert sind, dann - allgemein formuliert - die Sendeeinheit und die Empfangseinheit auf der gleichen Seite des Metallprodukts angeordnet sind. Ergänzend wird darauf hingewiesen, dass beim IMPOC-Messverfahren in der Regel zwei Messköpfe zum Einsatz kommen, die vorzugsweise identisch aufgebaut sind und auf entgegengesetzten Seiten des zu untersuchenden Metallprodukts angeordnet sind. Im Sinne der vorliegenden Erfindung können diese beiden Messköpfe jeweils als Messvorrichtung verstanden werden.

Gemäß einer vorteilhaften Weiterbildung der Erfindung kann im Hinblick auf eine Ausgestaltung des Gehäuses mit Öffnungen, denen die Sendeeinheit und Empfangseinheit zugeordnet sind, das Bewegen der Sendeeinheit und/oder der Empfangseinheit mittels der damit verbundenen Verstelleinrichtung derart erfolgen, dass die Sendeeinheit und/oder die Empfangseinheit durch die ihnen jeweils zugeordneten Öffnungen in das Gehäuse hinein bewegt werden oder aus dem Gehäuse heraus nach außen bewegt werden. Hierdurch wird wie vorstehend bereits erläutert ein Abstand zwischen einerseits der Sendeeinheit und/oder der Empfangseinheit und andererseits dem Metallprodukt auf einen vorbestimmten Wert eingestellt oder gezielt geändert. Falls die Sendeeinheit und/oder die Empfangseinheit der Messvorrichtung mittels der damit verbundenen Verstelleinrichtung in das Gehäuse hinein bewegt werden, kann dadurch ein vorteilhaft sehr kleiner Abstand dieser Komponente(n) der Messvorrichtung zum Metallprodukt erreicht werden.

Gemäß einer vorteilhaften Weiterbildung der Erfindung ist eine Verstelleinrichtung, mit der die Sendeeinheit und/oder die Empfangseinheit jeweils wirkverbunden ist und wie erläutert eine Bewegung dieser Komponenten der Messvorrichtung relativ zum Gehäuse der Vorrichtung realisiert wird, außerhalb des Gehäuses angeordnet. Ein großer Verstellweg für eine solche Verstelleinrichtung lässt sich in robuster Weise durch eine teleskopartige Ausgestaltung dieser Verstelleinrichtung erreichen.

Alternativ hierzu ist es auch möglich, zumindest eine Verstelleinrichtung für ein Bewegen der Sendeeinheit und/oder der Empfangseinheit auch innerhalb des Gehäuses vorzusehen.

Falls es sich bei dem Gehäuse um einen Ofen zur Wärmebehandlung des Metallprodukts handelt, ist in diesem Gehäuse in der Regel eine bestimmte Gas-Atmosphäre mit hoher Temperatur enthalten, die gegenüber der Umgebung des Gehäuses abzudichten ist. Zu diesem Zweck sind gemäß einer vorteilhaften Weiterbildung der Erfindung Abschirmungen vorgesehen, die sowohl eine Abdichtung des Innenraums des Gehäuses gegenüber der Außenumgebung sicherstellen und gleichzeitig in Bezug auf die Wellen der elektromagnetischen Strahlung, die von der Sendeeinheit erzeugt werden, durchlässig sind. Beispielsweise kann es sich bei solchen Abschirmungen um Fenster handeln. Jedenfalls wird in diesem Zusammenhang darauf hingewiesen, dass die Abschirmungen bzw. Fenster in Bezug auf die Strahlungswellen eines elektromagnetischen Felds und/oder in Bezug auf den verbleibenden und/oder resultierenden Teil der vorstehend erläuterten physikalischen Wechselwirkung die vorstehend genannte erforderliche Durchlässigkeit aufweisen. Damit ist es möglich, sowohl die Sendeeinheit als auch die Empfangseinheit jeweils auf einer Außenseite dieser Abschirmungen bzw. Fenster anzuordnen, die sich entgegengesetzt zum Innenraum des Gehäuses befindet.

In Bezug auf die Möglichkeit, dass das Gehäuse der Vorrichtung Teil eines Ofens zur Wärmebehandlung sein kann, wird an dieser Stelle auch darauf hingewiesen, dass von den Gasen, die in einem solchen Ofen enthalten sein können, beispielsweise Wasserstoff, Stickstoff oder Verbrennungsgase, ein Gefährdungspotenzial ausgeht. Entsprechend ist auch in dieser Hinsicht ein wirkungsvolles Abdichten der ersten und zweiten Öffnung, die in der Wandung des Gehäuses ausgebildet sind, gegenüber der Umgebung wichtig.

In vorteilhafter Weiterbildung der Erfindung sind die besagten Abschirmungen, mit denen im Bereich der Öffnungen des Gehäuses eine Abdichtung des Innenraums des Gehäuses gegenüber der Außenumgebung erreicht wird, derart beschaffen, dass mit diesen Abschirmungen auch eine Verminderung der Wärmestrahlung erreicht wird. Dies bedeutet, dass eine vergleichsweise hohe Temperatur innerhalb des Gehäuses durch diese Abschirmungen bzw. Fenster geeignet vermindert wird, mit der Folge, dass auf der entgegengesetzten Seite dieser Abschirmungen bzw. Fenster, auf der eine Komponente der Messvorrichtung, d.h. eine Sendeeinheit bzw. Empfangseinheit, angeordnet ist, eine verminderte Temperatur herrscht.

Wie vorstehend erläutert, sind im Bereich der ersten und zweiten Öffnung des Gehäuses jeweils Abschirmungen insbesondere in Form von Fenstern vorgesehen, mit denen neben einer Abdichtung der innerhalb des Gehäuses herrschenden Atmosphäre bzw. Gaszusammensetzung gegenüber der Umgebung auch eine Verminderung der thermischen Wärmestrahlung erreicht wird. Um eine noch bessere Beweglichkeit der Sendeeinheit und/oder der Empfangseinheit hinsichtlich eines möglichst geringen Abstands zum Metallprodukt zu erreichen, ist nach einer vorteilhaften Weiterbildung der Erfindung eine Abschirmung mit einer insbesondere elastisch verformbaren Dichteinrichtung verbunden, wobei die Dichteinrichtung mit den Rändern der ersten bzw. zweiten Öffnung befestigt ist und dadurch der Innenraum des Gehäuses gegenüber der Umgebung abgedichtet ist.

Die soeben genannte Dichteinrichtung ist mit geeigneten Verstellmitteln verbunden. Hiermit ist es möglich, die Dichteinrichtung zusammen mit der damit verbundenen Abschirmung (z.B. in Form eines Fensters) in das Gehäuse hineinzubewegen oder aus dem Gehäuse herauszubewegen. Zweckmäßigerweise sind diese Verstellmittel teleskopartig ausgebildet, wodurch sich in einfacher und insbesondere auch platzsparender Weise ein großer Verstellweg erreichen lässt.

Falls eine Dichteinrichtung, die an den Rändern der ersten Öffnung und der zweiten Öffnung angebracht ist, beispielsweise in das Gehäuse hineinbewegt worden ist, kann in Anpassung daran bzw. in gleichem Maße auch die zugeordnete Sendeeinheit/ Empfangseinheit ebenfalls in das Gehäuse hineinbewegt werden, um damit einen möglichst geringen Abstand zum Metallprodukt zu erreichen bzw. einen vorbestimmten Abstand zum Metallprodukt einzustellen oder gezielt zu verändern.

Die vorstehend genannte insbesondere flexible Dichteinrichtung kann in Form eines verformbaren Faltenbalgs ausgebildet sein.

Für die vorstehend genannte insbesondere flexible Dichteinrichtung kann eine Formgebung vorgesehen sein, so dass sie im Querschnitt rund, gerundet, oval, rechteckig oder quadratisch ausgebildet ist oder eine Kombination von diesen Formen aufweist.

In vorteilhafter Weiterbildung der Erfindung kann vorgesehen sein, dass die insbesondere flexible Dichteinrichtung an ihrer Stirnseite, die dem Innenraum des Gehäuses zugewandt ist, und/oder seitliche Abschnitte der Dichteinrichtung jeweils mit einer Schutzschicht und/oder einer Isolierung gegen elektromagnetische und/oder thermische Strahlung ausgestattet sind. Hiermit wird der Vorteil erreicht, dass eine solche Dichteinrichtung weniger empfindlich ist und eine größere Lebensdauer aufweist, wenn sie wie erläutert an den Rändern der ersten bzw. zweiten Öffnung des Gehäuses befestigt ist und somit in unmittelbarer Nähe zum Innenraum des Gehäuses, in dem sehr hohe Temperaturen herrschen können, angeordnet ist. Mutatis mutandis gilt dies auch in Bezug auf die elektromagnetische Strahlung, denen die Dichteinrichtung ausgesetzt sein kann.

Bei den wesentlichen Bestandteilen der Messvorrichtung, nämlich Sendeeinheit und Empfangseinheit, handelt es sich um empfindliche Komponenten, die insbesondere gegen die Einwirkung von zu hohen Temperaturen zu schützen sind. Aus diesem Grund werden diese Komponenten ohne Schutz nicht unmittelbar innerhalb des Gehäuses, in dem in der Regel sehr hohe Temperaturen herrschen, angeordnet oder darin eingebracht - stattdessen sind die Sendeeinheit und die Empfangseinheit von dem Innenraum des Gehäuses stets durch die Abschirmungen in Form von Fenstern getrennt, durch die es wie vorstehend erläutert auch zu einer Verminderung der Wärmestrahlung kommt.

Zum weiteren Schutz gegen hohe Temperaturen kann gemäß einer vorteilhaften Weiterbildung der Erfindung zumindest eine Kühleinrichtung vorgesehen sein. Diese Kühleinrichtung dient dazu, die im Bereich der ersten bzw. zweiten Öffnung des Gehäuses vorgesehenen Abschirmungen bzw. Fenster und/oder die Komponenten der Messvorrichtung (Sendeeinheit und/oder Empfangseinheit) und/oder die Dichteinrichtung zu kühlen. Zu diesem Zweck kann vorgesehen sein, dass die Kühleinrichtungen Kühlleitungen und/oder Kavitäten umfasst, die in einer Wandung des Gehäuses insbesondere angrenzend zum ersten Fenster und/oder zweiten Fenster ausgebildet sind und von einem Kühlfluid insbesondere in Form einer Flüssigkeit durchströmt werden. Ergänzend oder alternativ kann vorgesehen sein, dass Kühlleitungen und/oder Kavitäten der Kühleinrichtung in dem Material der Dichteinrichtung vorgesehen sind, und/oder dass Kühlleitungen insbesondere in Form von Leitungsschlangen an zumindest einer Komponente der Messvorrichtung (Sendeeinheit und/oder Empfangseinheit) vorgesehen sind.

Während des praktischen Einsatzes der vorliegenden Erfindung ist es weiterhin von Bedeutung, dass die Abschirmungen, in Form von Fenstern, frei bleiben von jeglicher Form von Staub, Verschmutzungen oder dergleichen. Zu diesem Zweck ist nach einer vorteilhaften Weiterbildung der Erfindung zumindest eine Spülgaseinrichtung vorgesehen, durch die ein Spülgas auf die Abschirmungen bzw. Fenster ausgebracht wird. Das Ausbringen von Spülgas kann durch geeignete Düsen der Spülgaseinrichtung erfolgen, die auf der Außenseite der Abschirmungen bzw. Fenster und/oder an deren Innenseite, d.h. im Innenraum des Gehäuses angeordnet sind. Somit wird die Oberfläche der Abschirmungen bzw. Fenster während des Einsatzes der Vorrichtung bzw. bei Durchführung des Verfahrens gemäß der vorliegenden Erfindung durch das Ausbringen des Spülgases vorzugsweise permanent "freigeblasen". In Folge dessen bleibt die Oberfläche dieser Abschirmungen bzw. Fenster im Wesentlichen frei von Ablagerungen in Form von Staub oder anderen Verschmutzungen.

Ergänzend darf an dieser Stelle darauf hingewiesen werden, dass insbesondere das Ausbringen von Spülgas auf eine Außenseite der Abschirmungen bzw. Fenster auch zu einer Kühlung dieser Abschirmungen bzw. Fenster beitragen kann.

Im Hinblick auf die zumindest eine Eigenschaft des Metallprodukts, die sich mittels der vorliegenden Erfindung ermitteln lässt, darf an dieser Stelle darauf hingewiesen werden, dass es sich bei der Eigenschaft des Metallprodukts um folgende Größen handeln kann:
- Mikrostruktur,
- Phasenanteil,
- Rekristallisationsgrad,
- Korngröße,
- Textur,
- Polfigur,
- Orientierungsverteilungsfunktion,
- Oxidationsschicht, und/oder
- mechanischer Kennwert des Metallprodukts.

In vorteilhafter Weiterbildung der Erfindung können auch mehrere Messvorrichtungen mit jeweils einer Sendeeinheit und Empfangseinheit zum Einsatz kommen. Für diesen Fall können in der Wandung des Gehäuses dann auch entsprechend große Öffnungen oder eine Mehrzahl von Öffnungen ausgebildet sein, denen die einzelnen Sendeeinheiten/Empfangseinheiten der jeweiligen Messvorrichtung zugeordnet sind.

In vorteilhafter Weiterbildung der Erfindung können die Öffnungen, die in der Wandung des Gehäuses ausgebildet sind und denen die jeweiligen Komponenten der Messvorrichtung zugeordnet sind, mit einem leistungsfähigen Isoliermaterial ausgestattet sein. Gleiches gilt auch für die Engstelle, die in der Wandung des Gehäuses im Bereich der ersten und/oder zweiten Öffnung ausgebildet sein kann. Jedenfalls wird durch ein solches Isoliermaterial der Vorteil erreicht, dass auf eine Dichteinrichtung, die mit den Rändern einer jeweiligen Öffnung befestigt sein kann, und/oder auf eine Komponente der Messvorrichtung (Sendeeinheit und/oder Empfangseinheit), die wie vorstehend erläutert im Bereich einer solchen Öffnung oder angrenzend hierzu relativ zur Wandung des Gehäuses bewegt werden kann, eine geringere thermische Strahlung einwirkt.

Der Einsatz der vorliegenden Erfindung eignet sich insbesondere bei der Wärmebehandlung eines Metallprodukts, d.h. in Verbindung mit einer Ofeneinrichtung, durch welches das Metallprodukt im Zuge seiner Wärmebehandlung hindurchgeführt wird. Hierbei ist es dann mit Hilfe der vorliegenden Erfindung möglich, "Online" bzw. in Echtzeit zumindest einer Eigenschaft des Metallprodukts zu ermitteln. Im Zuge dessen ist es auch möglich, auf Grundlage des Messwertes für die zumindest eine Eigenschaft des Metallprodukts dessen Herstellungsprozess vorzugsweise mit einer Regelstrecke zu beeinflussen.

Nachstehend sind bevorzugte Ausführungsbeispiele der Erfindung anhand einer schematisch vereinfachten Zeichnung im Detail beschrieben.

Es zeigen:
- Fig. 1-4: mögliche Ausführungsformen für ein Gehäuse einer erfindungsgemäßen Vorrichtung,
- Fig. 5: eine Längsschnittansicht eines Gehäuses einer erfindungsgemäßen Vorrichtung,
- Fig. 6: eine vergrößerte Teil-Längsschnittansicht der Darstellung von Fig. 2,
- Fig. 7: eine Schnittansicht durch eine Abschirmung, die an einer Wandung des Gehäuses der erfindungsgemäßen Vorrichtung oder an einer hierzu verwendeten Dichteinrichtung befestigt ist,
- Fig. 8: eine Schnittansicht durch eine Abschirmung der Fig. 7,
- Fig. 9-12: jeweils Längsschnittansichten durch eine erfindungsgemäße Vorrichtung gemäß weiterer Ausführungsformen, mit einem Gehäuse gemäß Fig. 2,
- Fig. 13: eine Perspektivansicht durch das Gehäuse einer erfindungsgemäßen Vorrichtung gemäß einer weiteren Ausführungsform,
- Fig. 14: eine Perspektivansicht durch das Gehäuse einer erfindungsgemäßen Vorrichtung gemäß einer weiteren Ausführungsform,
- Fig. 15: eine vereinfachte Seitenansicht einer erfindungsgemäßen Vorrichtung gemäß einer weiteren Ausführungsform,
- Fig. 16: eine Schnittansicht durch ein Gehäuse einer erfindungsgemäßen Vorrichtung gemäß einer Ausführungsform, bei der die Komponenten einer zugehörigen Messvorrichtung auf der gleichen Seite des Gehäuses angeordnet sind,
- Fig. 17-19: verschiedene Ansichten von Details einer erfindungsgemäßen Vorrichtung gemäß einer noch weiteren Ausführungsform,
- Fig. 20: eine Querschnittsansicht durch das Gehäuse eine erfindungsgemäßen Vorrichtung gemäß einer Variante zu Fig. 19,
- Fig. 21-22: verschiedene Ansichten von Details einer erfindungsgemäßen Vorrichtung gemäß einer noch weiteren Ausführungsform, und
- Fig. 25: eine Längsschnittansicht durch ein Gehäuse einer erfindungsgemäßen Vorrichtung gemäß einer noch weiteren Ausführungsform.

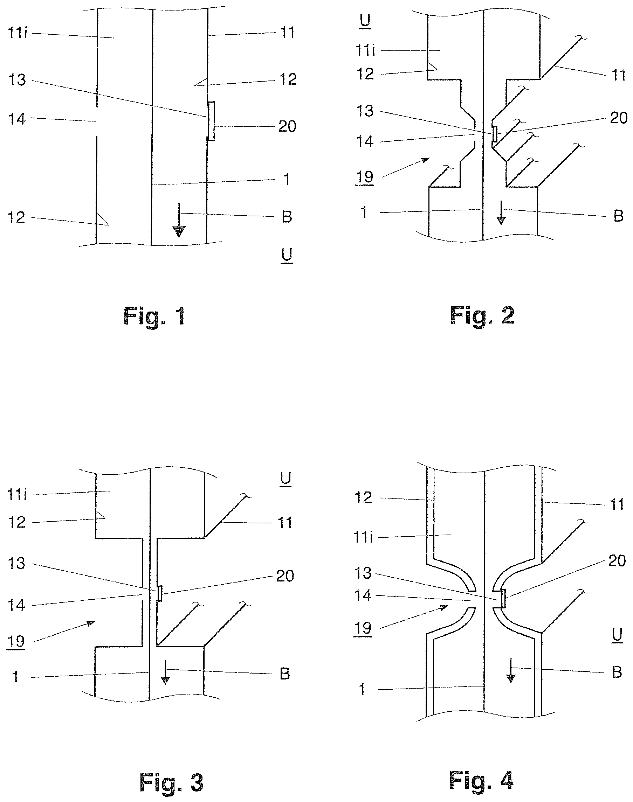

Nachstehend sind unter Bezugnahme auf die Fig. 1-25 bevorzugte Ausführungsformen einer Vorrichtung 10 und eines entsprechenden Verfahrens gemäß der vorliegenden Erfindung dargestellt und erläutert, um damit zumindest eine Eigenschaft eines Metallprodukts 1 während seiner metallurgischen Herstellung zu ermitteln. Gleiche Merkmale in der Zeichnung sind jeweils mit gleichen Bezugszeichen versehen. An dieser Stelle wird gesondert darauf hingewiesen, dass die Zeichnung lediglich vereinfacht und insbesondere ohne Maßstab dargestellt ist.

Die erfindungsgemäße Vorrichtung 10 umfasst ein Gehäuse 11 mit einem Innenraum 11i. Ein Metallprodukt 1, beispielsweise in Form eines Kalt- oder Warmbands, kann durch den Innenraum 11i des Gehäuses 11 hindurchgeführt werden. Die Bewegungsrichtung, in der das Metallprodukt 1 durch das Gehäuse 11 hindurchgeführt wird, ist in der Zeichnung jeweils mit "B" bezeichnet und durch einen Pfeil oder ein entsprechendes Symbol kenntlich gemacht.

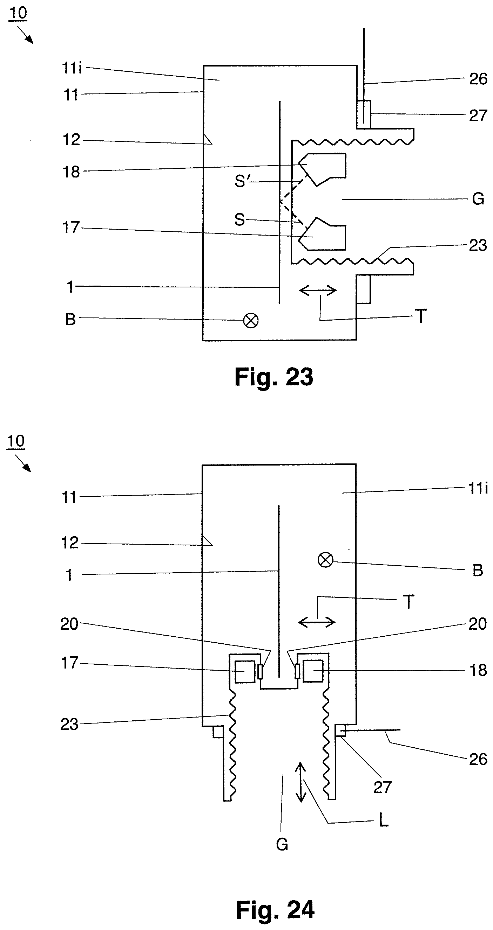

In einer Wandung 12 des Gehäuses 11 der Vorrichtung 10 sind zumindest eine erste Öffnung 13 und eine zweite Öffnung 14 ausgebildet. Falls diese beiden Öffnungen 13, 14 auf der gleichen Seite des Gehäuses 11 ausgebildet sind, können diese Öffnungen 13, 14 ggf. auch zu einer gemeinsamen Öffnung G zusammengefasst sein, was nachfolgend noch gesondert erläutert ist (vgl. Fig. 23, Fig. 24).

Im Bereich der Öffnungen 13, 14 des Gehäuses 11 der Vorrichtung 10 sind jeweils Abschirmungen 20 angeordnet, die beispielsweise in Form von Fenstern ausgebildet sind. Ohne hierin eine Beschränkung zu sehen, werden diese Abschirmungen nachfolgend stets nur als "Fenster" 20 bezeichnet. Diese Fenster 20 sind Teil eines Abdichtungs-Konzepts gemäß der vorliegenden Erfindung, um damit eine Abdichtung des Innenraums 11i des Gehäuses 11 gegenüber der Umgebung U zu erreichen.

Die Ausführungsform von Fig. 1 zeigt eine Längsschnittansicht durch das Gehäuse 11 einer erfindungsgemäßen Vorrichtung. Wie ersichtlich, wird hierbei ein Metallprodukt 1 entlang einer Bewegungsrichtung B durch das Gehäuse 11 hindurch bewegt (in der Zeichenebene von Fig. 1 von oben nach unten). Der Umgebungsbereich des Gehäuses 11 bzw. dessen Außenseite ist symbolisch durch "U" bezeichnet. Das erste Fenster 13 und das zweite Fenster 14 sind hierbei in der Wandung 12 des Gehäuses auf entgegengesetzten Seiten des Metallprodukts 1 ausgebildet. Die Fenster 20 sind hierbei direkt an den Rändern der jeweiligen Öffnung 13, 14 bzw. innerhalb dieser Öffnungen befestigt, wobei zur Vereinfachung und aus Gründen der besseren Veranschaulichung das Fenster 20 für die zweite Öffnung 14 in Fig. 1 nicht gezeigt ist.

Die Ausführungsformen gemäß der Fig. 2-4 unterscheiden sich von der Ausführungsform von Fig. 1 dadurch, dass hierbei das Gehäuse 11 im Bereich der ersten und zweiten Öffnung 13, 14 eine Engstelle 19 aufweist. Ansonsten unterscheiden sich die Ausführungsformen gemäß der Fig. 2-4 lediglich durch die Geometrie der Engstelle 19, so dass zur Vermeidung von Wiederholungen auf die Erläuterung zu Fig. 1 verwiesen werden.

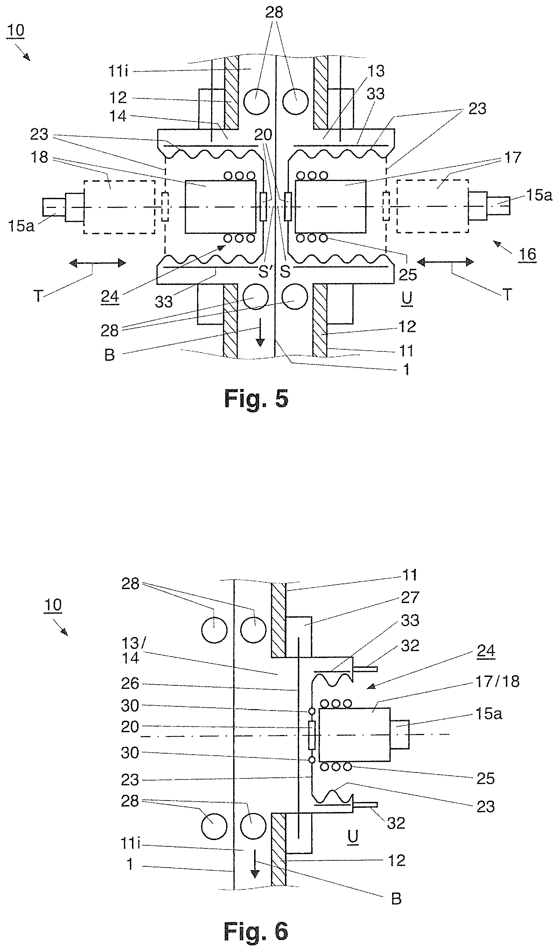

Die Vorrichtung 10 umfasst zumindest eine Messvorrichtung 16 (vgl. Fig. 5), die aus einer Sendeeinheit 17 und einer Empfangseinheit 18 besteht.

Die Komponenten der Messvorrichtung 16, d.h. die Sendeeinheit 17 und die Empfangseinheit 18, sind jeweils mit einer Verstelleinrichtung 15a wirkverbunden. Bei einer Aktuierung der jeweiligen Verstelleinrichtung 15a können die Sendeeinheit 17 bzw. Empfangseinheit 18 relativ zur Wandung 12 des Gehäuses bewegt werden.

Unter Bezugnahme auf die Fig. 5, die eine Längsschnittansicht durch das Gehäuse 11 der erfindungsgemäßen Vorrichtung 10 zeigt, werden nun weitere Einzelheiten für die Sendeeinheit 17 bzw. Empfangseinheit 18 und den Funktionsmechanismus in Bezug auf deren mögliche Bewegung relativ zur Wandung 12 des Gehäuses 11 erläutert.

Bei der Ausführungsform von Fig. 5 kann ein Metallprodukt 1 durch das Gehäuse 11 hindurch in einer Bewegungsrichtung B bewegt werden, nämlich in der Zeichenebene von Fig. 5 von oben nach unten. Die erste Öffnung 13 und die zweite Öffnung 14 sind in der Wandung 12 des Gehäuses derart ausgebildet, dass sie sich auf entgegengesetzten Seiten des Metallprodukts 1 befinden.

In Bezug auf die beiden Öffnungen 13, 14, die in der Wandung 12 des Gehäuses 11 ausgebildet sind, versteht sich, dass die Sendeeinheit 17 der ersten Öffnung 13 zugeordnet ist, wobei die Empfangseinheit 18 der zweiten Öffnung 14 zugeordnet ist.

Entsprechend kann die Sendeeinheit 17 durch die erste Öffnung 13 hindurch oder angrenzend hierzu entweder hinein in den Innenraum 11i des Gehäuses 11 bewegt werden (in der Zeichenebene der Fig. 5 von rechts nach links), oder in die Gegenrichtung hierzu, nämlich heraus aus dem Innenraum 11i in Richtung nach außen (in der Zeichenebene der Fig. 5 von links nach rechts). Wie vorstehend bereits genannt, ist die Sendeeinheit 17 mit einer Verstelleinrichtung 15a wirkverbunden, die bei der hier in Fig. 5 gezeigten Darstellung an einer äußeren Stirnseite der Sendeeinheit 17 angebracht und vorzugsweise teleskopartig ausgebildet ist. Durch eine Aktuierung der Verstelleinrichtung 15a kann somit die Sendeeinheit 17 entweder in den Innenraum 11i des Gehäuses 11 hinein oder aus dem Gehäuse 11 heraus bewegt werden.

In gleicher Weise ist die Empfangseinheit 18 an ihrer äußeren Stirnseite ebenfalls mit einer insbesondere teleskopartig ausgebildeten Verstelleinrichtung 15a verbunden. Bei einer Aktuierung dieser Verstelleinrichtung 15a kann die Empfangseinheit 18 durch die zweite Öffnung 14 hindurch oder angrenzend hierzu entweder in den Innenraum 11i des Gehäuses 11 hinein oder in die Gegenrichtung, nämlich aus dem Gehäuse 11 heraus nach außen bewegt werden.

An den Rändern der ersten Öffnung 13 und der zweiten Öffnung 14 sind jeweils Dichteinrichtungen 23 befestigt, die jeweils aus einem elastisch verformbaren Material gebildet sind. Beispielsweise können diese Dichteinrichtungen 23 aus einem sog. Faltenbalg gebildet sein. Für die nachfolgende Beschreibung wird eine solche Dichteinrichtung 23 lediglich kurz als "Faltenbalg" bezeichnet, ohne dass hierin eine Beschränkung zu sehen ist.

In Folge einer Befestigung eines jeweiligen Faltenbalgs 23 an den Rändern der jeweiligen Öffnungen 13, 14 wird der Innenraum 11i des Gehäuses 11 an der Stelle dieser Öffnungen 13, 14 gegenüber seiner äußeren Umgebung durch die Faltenbälge 23 abgedichtet.

Jeder Faltenbalg 23 ist jeweils mit einer Stützstruktur 33 versehen, die entlang einer Längserstreckung des Faltenbalgs 23 (d.h. Zeichenebene von Fig. 5 jeweils horizontal) verläuft. Durch eine solche Stützstruktur 33 wird ein Faltenbalg 23 in Bezug auf seine seitlichen Wandlungen insbesondere dann stabilisiert, wenn der Faltenbalg 23 beispielsweise weit in den Innenraum 11i des Gehäuses hinein bewegt ist, wie es beispielsweise in der Darstellung von Fig. 5 gezeigt ist. Diesbezüglich wird darauf hingewiesen, dass in den Darstellungen von Fig. 5 und 6 diese Stützstrukturen 33 für die Faltenbälge 23 zur Vereinfachung lediglich symbolisch durch horizontale Linien dargestellt sind.

Fig. 6 zeigt eine Teil-Längsschnittansicht der Darstellung von Fig. 5 und verdeutlicht weitere Einzelheiten hiervon. Im Hinblick auf eine Symmetrie der Anordnung von Bauteilen an der ersten Öffnung 13 bzw. der zweiten Öffnung 14 ist in der Fig. 6 zur Vereinfachung nur eine Seite des Gehäuses 11 gezeigt. Die Darstellung von Fig. 6 gilt in gleicher Weise für die erste Öffnung 13 sowie für die zweite Öffnung 14, was auch dadurch zum Ausdruck kommen, dass die in Fig. 6 gezeigte Komponente der Messvorrichtung 16 mit "17" oder "18" bezeichnet ist, weil es sich hierbei entweder um die Sendeeinheit 17 oder um die Empfangseinheit 18 handelt.

Eine äußere Stirnseite des Faltenbalgs 23 ist mit Verstellmitteln 32 verbunden, mit denen eine vorzugsweise translatorische Verstellung des Faltenbalgs 23 möglich ist, nämlich entweder hinein in den Innenraum 11i des Gehäuses 11 oder in die Gegenrichtung, d.h. heraus aus dem Gehäuse 11. In diesem Zusammenhang wird darauf hingewiesen, dass ein Bewegen bzw. Verstellen eines jeweiligen Faltenbalgs 23 durch die damit verbundenen Verstellmittel 32 unabhängig von der Verstelleinrichtung 15a bzw. einem Bewegen einer Komponente der Messvorrichtung 16 (Sendeeinheit 17 und/oder Empfangseinheit 18) erfolgen kann.

Bei der Ausführungsform von Fig. 5 bzw. Fig. 6 ist an einer dem Innenraum 11i des Gehäuses 11 zugewandten Stirnseite der einzelnen Faltenbälge 23 jeweils ein Fenster 20 integriert. Bei einem Verstellen eines Faltenbalgs 23 wird somit ein darin integriertes Fenster 20 gleichzeitig mitbewegt. Somit handelt es sich bei den Fenstern 20 gemäß der Ausführungsform von Fig. 5 bzw. Fig. 6 um "wandernde Fenster", deren jeweilige Beabstandung zum Metallprodukt 1 von der jeweiligen Positionierung des zugehörigen Faltenbalgs 23 abhängig ist.

Wie vorstehend bereits erläutert, ist die Sendeeinheit 17 der ersten Öffnung 13 zugeordnet, wobei die Empfangseinheit 18 der zweiten Öffnung 14 zugeordnet ist. In Folge dessen kann die Sendeeinheit 17 beispielsweise durch die erste Öffnung 13 mittels einer Aktuierung der zugeordneten Verstelleinrichtung 15a in den Innenraum 11i des Gehäuses 11 hinein bewegt werden. Synchronisiert zu dieser Bewegung der Sendeeinheit 17 versteht sich, dass zuvor auch der Faltenbalg 23 auf der rechten Seite des Metallprodukts 1, welcher an den Rändern der ersten Öffnung 13 befestigt ist und dadurch die Sendeeinheit 17 entlang ihres Außenumfangs umschließt, durch eine Aktuierung der Verstellmittel 32 in den Innenraum 11i des Gehäuses 11 hineinbewegt worden ist. Diese Positionen sowohl für den Faltenbalg 23 als auch für die Sendeeinheit 17 auf der rechten Seite des Metallprodukts 1 sind in der Fig. 5 jeweils durch Volllinien dargestellt.

In gleicher Weise wie die Sendeeinheit 17 ist bei der Darstellung von Fig. 5 auch die Empfangseinheit 18 in den Innenraum 11i des Gehäuses 11 hinein bewegt, nämlich durch eine Aktuierung der damit verbundenen Verstelleinrichtung 15a. Hiermit einher geht auch, dass zuvor oder zumindest gleichzeitig der Faltenbalg 23, der auf der linken Seite des Metallprodukts 1 angeordnet ist und die Empfangseinheit 18 entlang ihres Außenumfangs umschließt, durch eine Aktuierung der damit verbundenen Verstellmittel 32 in den Innenraum 11i des Gehäuses 11 hineinbewegt worden ist. Diese Positionen sowohl für den Faltenbalg 23 als auch für die Empfangseinheit 18 auf der linken Seite des Metallprodukts 1 sind in der Fig. 5 ebenfalls jeweils durch Volllinien gezeigt.

Die Positionen, in denen bei der Darstellung von Fig. 5 sowohl die Sendeeinheit 17 als auch die Empfangseinheit 18 jeweils mit Volllinien gezeigt sind, entsprechen beispielsweise einer Betriebsposition dieser beiden Komponenten der Messvorrichtung 16, in denen sie möglichst nahe an das Metallprodukt 1 heranbewegt worden sind. Anders ausgedrückt, ist für an diesen Positionen zwischen einerseits der Sendeeinheit 17 bzw. Empfangseinheit 18 und andererseits dem bandförmigen Metallprodukt 1 ein vorbestimmter Abstand eingestellt, der damit einen möglichst kleinen Wert annimmt, beispielsweise von etwa 10 mm.

Die Positionierung der Sendeeinheit 17 bzw. Empfangseinheit 18 gemäß der Darstellung von Fig. 5 verdeutlicht, dass diese Komponenten der Messvorrichtung 16 nicht unmittelbar innerhalb des Innenraums 11i des Gehäuses 11 angeordnet sind, sondern dass sich zwischen ihnen und dem Metallprodukt 1 stets die Stirnseite eines zugeordneten Faltenbalgs 23 mit einem darin integrierten Fenster 20 befindet.

Bei dem Metallprodukt 1, welches bei der Ausführungsform von Fig. 5 bzw. Fig. 6 durch das Gehäuse 11 der Vorrichtung 10 entlang der Bewegungsrichtung B hindurchgeführt wird, kann es sich um ein bandförmiges Material handelt, beispielsweise um Kaltband oder Warmband. Unter Berücksichtigung dessen wird darauf hingewiesen, dass ein Bewegen der Komponenten der Messvorrichtung 16 bei der Fig. 5 jeweils in transversaler Richtung erfolgt, d.h. in einer Richtung orthogonal zu einer Oberfläche des bandförmigen Metallprodukts 1. Eine solche Bewegungsrichtung ist in der Fig. 5 durch einen Doppelpfeil symbolisiert, der mit "T" bezeichnet ist.

An dieser Stelle wird gesondert darauf hingewiesen, dass die beiden Verstelleinrichtungen 15a, die jeweils mit der Sendeeinheit 17 und der Empfangseinheit 18 wirkverbunden sind, unabhängig voneinander aktuiert werden können. In Folge dessen ist es möglich, dass diese Komponenten 17, 18 der Messvorrichtung 16 auch unabhängig voneinander Richtung in transversaler Richtung T bewegt werden können, um in den Innenraum 11i des Gehäuses 11 hinein oder aus dem Gehäuse 11 heraus bewegt werden.

In der Darstellung von Fig. 5 sind durch jeweils strichlierte Linien sowohl für die Komponenten der Messvorrichtung 16, d.h. die Sendeeinheit 17 und die Empfangseinheit 18, als auch für die zugeordneten Faltenbälge 23 Positionen angedeutet, in denen diese Elemente aus der ersten Öffnung 13 bzw. der zweiten Öffnung 14 herausbewegt worden sind. In Bezug auf die Faltenbälge 23 wird eine solche Bewegung zwischen den Positionen, die in der Fig. 5 einerseits durch Volllinien und andererseits durch die strichlierten Linien gezeigt sind, dadurch ermöglicht, dass diese Faltenbälge 23 aus einem elastisch verformbaren Material gebildet sind.

Die Fig. 6 zeigt die Sendeeinheit 17 bzw. die Empfangseinheit 18 in einer Position, in der sie durch eine Aktuierung der jeweils zugeordneten Verstelleinrichtung 15a in transversaler Richtung T aus dem Innenraum 11a heraus bewegt worden ist. In gleicher Weise ist hierbei der Faltenbalg 23 nach außen bewegt worden, so dass sich das in der Stirnseite des Faltenbalgs 23 integrierte Fenster weiterhin angrenzend zur inneren Stirnseite der Sendeeinheit 17 bzw. Empfangseinheit 18 befindet.

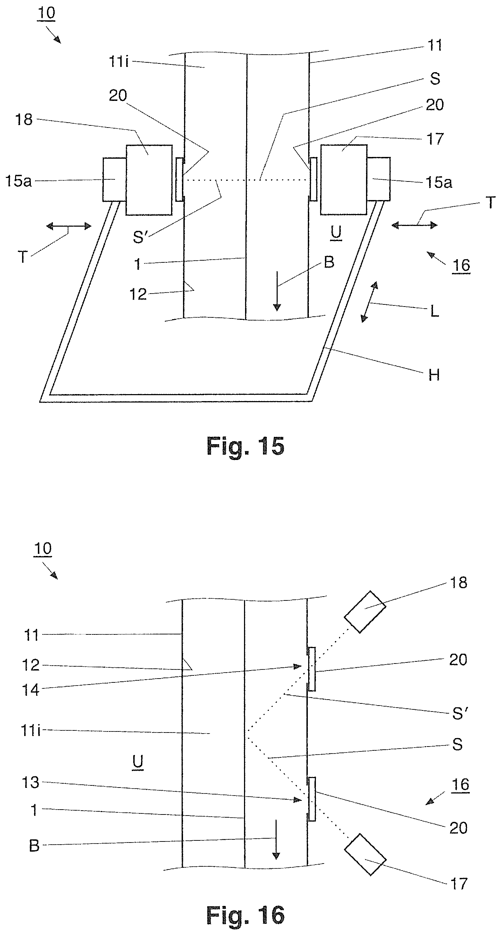

Angrenzend zu den beiden Öffnungen 13, 14 ist an dem Gehäuse 11 jeweils eine Verschlusseinrichtung 26 angebracht, die in einem zugeordneten Behälter 27 aufgenommen bzw. darin verschieblich geführt ist. Falls sich die Komponenten der Messvorrichtung 16 (= Sendeeinheit 17 und Empfangseinheit 18) jeweils in der Position von Fig. 6 befinden, kann mittels der Verschlusseinrichtung 26 ein Verschließen der Öffnungen 13, 14, so wie es in der Fig. 6 dargestellt ist, und somit ein Abdichten des Innenraums 11 des Gehäuses gegenüber der Umgebung U erreicht werden. In Folge dessen ist es möglich, beispielsweise den Faltenbalg 23 von der Öffnung 13, 14 zu demontieren und dann die Sendeeinheit 17 bzw. die Empfangseinheit 18 weiter von dem Gehäuse 11 zu entfernen, etwa für Reparatur- und/oder Wartungszwecke, und/oder auch zur Durchführung einer Kalibrierung oder Vermessungen von externen Proben, die nicht in der eigentlichen Linie zur Herstellung des Metallprodukts 1 verarbeitet bzw. behandelt werden. Hierzu wird auch auf die Fig. 15 verwiesen, die nachfolgend noch gesondert erläutert ist.

Ausweislich der Darstellung von Fig. 6 können in dem Material des Faltenbalgs 23 an einer Stirnseite davon, die dem Innenraum 11i zugewandt ist, und vorzugsweise angrenzend zum Fenster 20 Kühlleitungen 30 integriert sein. Indem ein Kühlfluid, vorzugsweise in Form einer Kühlflüssigkeit, durch diese Kühlleitungen 30 hindurch geleitet wird, wird die Stirnseite des Faltenbalgs 23 geeignet gekühlt. Diese Kühlung an den Stirnseiten eines jeweiligen Faltenbalgs 23 kühlt zunächst ein darin integriertes Fenster 20 und kann auch zur Folge haben, dass die thermische Belastung in Folge von Wärmestrahlung, die auf die an der entgegengesetzten Seite des Fensters befindliche Komponente der Messvorrichtung 16 (d.h. Sendeeinheit 17 bzw. Empfangseinheit 18) einwirkt, vermindert ist.

Eine weitere Verminderung der thermischen Belastung für die Sendeeinheit 17 bzw. Empfangseinheit 18 kann dadurch erreicht werden, dass hierfür jeweils eine Kühleinrichtung 24 vorgesehen ist, durch die ein Kühlmittel, vorzugsweise eine Kühlflüssigkeit, zum Kühlen der entsprechenden Komponente der Messvorrichtung 16 geleitet wird. Bei der Darstellung von Fig. 5 bzw. Fig. 6 kann eine solche Kühleinrichtung 24 in Form einer Leitungsschlange 25 ausgebildet sein, die an einer Außenumfangsfläche der Sendeeinheit 17 bzw. Empfangseinheit 18 angebracht ist.

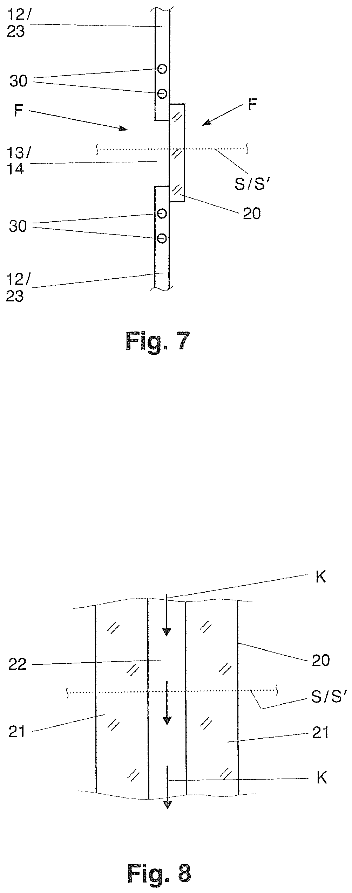

Weitere Maßnahmen zur Kühlung sind nachfolgend in den Fig. 7 und 8 erläutert.

Die Fig. 7 zeigt eine Schnittansicht (im Längsschnitt oder im Querschnitt) durch ein Fenster 20, das an einer Wandung 12 des Gehäuses 11 oder an einem Faltenbalg 23 befestigt sein kann. In der Wandung 12 des Gehäuses 11 bzw. in dem Material des Faltenbalgs 23 sind Kühlleitungen 30 und/oder Kavitäten 30 ausgebildet, durch die ein Kühlfluid, vorzugsweise eine Kühlflüssigkeit geleitet wird. Hierdurch wird erreicht, dass das Material der Wandung 12 bzw. eines Faltenbalgs 23 zumindest unmittelbar angrenzend an das Fenster 20 gekühlt wird, wodurch auch die Temperaturbelastung für dieses Fenster 20 und auch dessen Temperatur selber vermindert wird.

Fig. 8 zeigt einen Schnitt durch ein Fenster 20 und verdeutlicht eine doppelwandige Struktur, mit dem dieses Fenster 20 ausgebildet ist. Anders ausgedrückt, besteht dieses Fenster aus zumindest zwei Flächenelementen, die voneinander beabstandet sind und zwischen sich eine Kavität 22 einschließen. Durch diese Kavität 22 kann ein Kühlfluid, vorzugsweise ein Kühlgas geleitet werden, wodurch eine weitere Kühlung des Fensters 20 und damit auch eine Verminderung der Wärmestrahlung, die aus dem Innenraum 11i des Gehäuses 11 durch das Fenster 20 nach aussen gelangt, erreicht wird.

Die Vorrichtung 10 umfasst auch eine Spülgaseinrichtung mit Düsen, aus denen ein Spülgas auf die Oberfläche eines Fensters 20 ausgebracht werden kann. Dies ist in der Fig. 7 symbolisch durch die beiden Pfeile angedeutet, die jeweils mit "F" bezeichnet sind. Die Düsen der Spülgaseinrichtung können im Innenraum 11i des Gehäuses 11 und/oder an der Außenseite des Gehäuses 11, vorzugsweise angrenzend an die erste Öffnung 13 bzw. die zweite Öffnung 14, angeordnet sein.

In Bezug auf den Funktionsmechanismus, nach dem gemäß der Darstellung in den Fig. 5 und 6 ein Bewegen der Komponenten der Messvorrichtung 16 in transversaler Richtung T möglich ist, versteht sich, dass dieser Funktionsmechanismus mit der zugeordneten Anordnung von Sendeeinheit 17 und Empfangseinheit 18 im Bereich der jeweiligen Öffnungen 13, 14 für eine der Gehäuseformen möglich ist, die in den Fig. 1-4 gezeigt sind. Beispielsweise darf im Hinblick auf eine der Fig. 2-4 hervorgehoben werden, dass eine Anordnung der Komponenten der Messvorrichtung 16 gemäß der Darstellung von Fig. 5 und Fig. 6 insbesondere auch im Bereich einer Engstelle 19 des Gehäuses 11 möglich ist, an der die beiden Öffnungen 13, 14 ausgebildet sind.

Die Erfindung funktioniert unter Bezugnahme auf die Fig. 5 wie folgt:
Während seiner metallurgischen Herstellung wird das Metallprodukt 1 entlang der Bewegungsrichtung B durch das Gehäuse 11 der Vorrichtung 10 hindurchgeführt. In einer Betriebsstellung der Vorrichtung 10 sind Sendeeinheit 17 und Empfangseinheit 18, und synchronisiert hierzu auch die zugeordneten Faltenbälge 23, jeweils in transversaler Richtung T in den Innenraum 11i des Gehäuses 11 hinein bewegt, so wie es in der Fig. 5 jeweils durch Volllinien gezeigt und vorstehend bereits erläutert worden ist. Hierbei sind die Faltenbälge 23 mit den darin integrierten Fenstern 20 möglichst nah an das Metallprodukt 1 herangefahren, so dass die Sendeeinheit 17 und die Empfangseinheit 18 in einem vorbestimmten und ebenfalls möglichst geringen Abstand zum Metallprodukt 1, beispielsweise etwa 10 mm, angeordnet sein können.

Die Sendeeinheit 17 ist anlagentechnisch derart ausgebildet, dass von ihr ein elektromagnetisches Feld erzeugt wird beispielsweise in Form von Röntgenstrahlung. Die resultierende elektromagnetische Strahlung, in der Fig. 5 mit S bezeichnet, tritt von der Sendeeinheit 17 zunächst durch das Fenster 20 des zugeordneten Faltenbalgs 23 und anschließend durch das Metallprodukt 1 hindurch. In der Fig. 1 ist mit S' das Wellenmuster bezeichnet, welches nach dem Hindurchtreten durch das Metallprodukt 1 an dessen anderen Seite verbleibt und/oder resultiert und nach dem Hindurchtreten durch das Fenster 20 des Faltenbalgs 23, der die Empfangseinheit 18 umgibt, dann von der Empfangseinheit 18 empfangen wird.

Falls die Sendeeinheit 17 und die Empfangseinheit 18 der Vorrichtung 10 gemäß der Darstellung von Fig. 5 auf entgegengesetzten Seiten des Metallprodukts 1 angeordnet sind, kann das erfindungsgemäße Ermitteln von zumindest einer Eigenschaft des Metallprodukts 1 beispielsweise im Fall von Röntgenstrahlung, auf dem Transmissions- bzw. Durchstrahlprinzip beruhen.

Gemäß einer alternativen Ausführungsform ist es auch möglich, dass es sich bei den Komponenten 17, 18 von Fig. 5 jeweils um IMPOC-Messköpfe handeln kann.

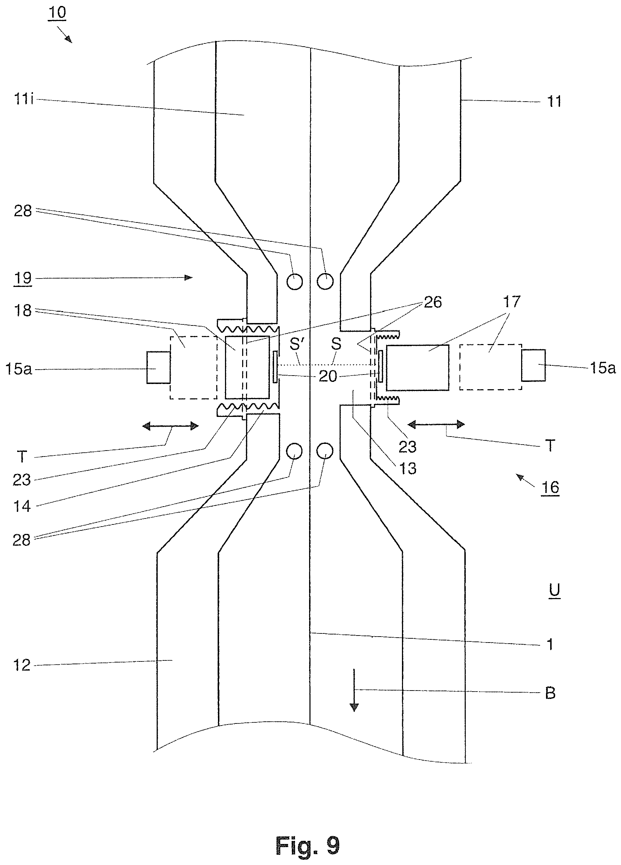

Dies gilt in gleicher Weise auch für die Ausführungsformen nach den Fig. 9-15 und Fig. 19 und Fig. 24, die nachstehend noch erläutert sind.

An dieser Stelle darf nochmals darauf hingewiesen werden, dass die Fenster 20, welche in die jeweiligen Faltenbälge 23 integriert sind, in Bezug auf die Wellen der elektromagnetischen Strahlung, vorzugsweise Röntgenstrahlung oder Laserstrahlung, oder in Bezug auf die Feldlinien des von der Sendeeinheit erzeugten elektromagnetischen Felds durchlässig sind.

Die Empfangseinheit 18 ist signaltechnisch mit einer (nicht gezeigten) Auswerteeinheit verbunden. Mittels dieser Auswerteeinheit wird dann der verbleibende und/oder resultierende Teil der physikalischen Wechselwirkung, beispielsweise in Form eines Wellenmusters der durchgelassenen Röntgenstrahlung, der von der Empfangseinheit 18 empfangen bzw. detektiert worden ist, geeignet ausgewertet und auf Grundlage dessen zumindest eine Eigenschaft bzw. ein Werkstoffparameter für das Metallprodukt 1 bestimmt.

Die vorliegende Erfindung zeichnet sich dadurch aus, dass auch während der metallurgischen Herstellung des Metallprodukts 1 ein Abstand zwischen den Komponenten (= Sendeeinheit 17 und/oder Empfangseinheit 18) der Messvorrichtung 16 und dem Metallprodukt 1 durch eine Bewegung der Sendeeinheit 17 und/oder der Empfangseinheit 18 in transversaler Richtung T auf einen vorbestimmten Abstand eingestellt oder gezielt werden kann. Beispielsweise kann dies in Anpassung an eine geänderte Dicke des Metallprodukts 1 erfolgen.

An dieser Stelle wird darauf hingewiesen, dass der zur Fig. 5 erläuterte Funktionsmechanismus, mit dem die Komponenten 17, 18 der Messvorrichtung angrenzend zu den Öffnungen 13, 14 des Gehäuses 11 unter Verwendung der ggf. durch den Einsatz der Stützstrukturen 33 stabilisierten Faltenbälge 23 bewegbar sind, auch bei anderen Ausführungsformen der erfindungsgemäßen Vorrichtung 10 zum Einsatz kommen kann.

Nachfolgend sind weitere Merkmale und Ausführungsformen der Vorrichtung 10 erläutert:
Die Vorrichtung 10 umfasst eine Schutz-Gleiteinrichtung 28 (vgl. Fig. 5, Fig. 6), die innerhalb des Innenraums 11i des Gehäuses 11 angeordnet ist. Diese Schutz-Gleiteinrichtung 28 erfüllt eine Sicherheitsfunktion und gewährleistet eine ausreichende Beabstandung des Metallprodukts 1, wenn dieses in der Bewegungsrichtung B durch das Gehäuse 11 hindurchgeführt wird, von den Öffnungen 13, 14 und/oder von den Komponenten der Messvorrichtung 16 und/oder von den Faltenbälgen 23. Beispielsweise wird mittels der Schutz-Gleiteinrichtung 28 verhindert, dass das Metallprodukt 1 in Folge einer möglichen seitlichen Bewegung in Richtung einer der Seitenwände des Gehäuses 11 in Kontakt mit den Stirnseiten der jeweiligen Faltenbälge 23 und der darin integrierten Fenster 20 gelangen kann. Entsprechend kann eine mögliche Beschädigung der Faltenbälge 23 und der dahinter angeordneten Komponenten der Messvorrichtung 16 durch das Metallprodukt 1 dank der Schutz-Gleiteinrichtung 28 verhindert werden.

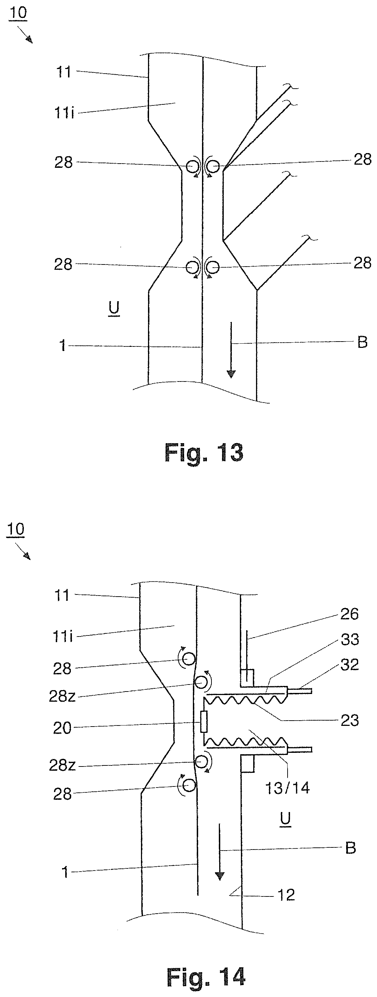

Die Ausführungsform von Fig. 13 verdeutlicht, dass die Schutz-Gleiteinrichtung jeweils Führungsrollen aufweist, die drehbar gelagert sind und in Kontakt mit dem Metallprodukt 1 sein können, währenddessen das Metallprodukt 1 in der Bewegungsrichtung B durch das Gehäuse 11 hindurchgeführt wird. Durch diesen Rollkontakt zwischen den Führungsrollen 28 und dem Metallprodukt 1 wird eine definierte Führung für das Metallprodukt 1 innerhalb des Innenraums 11i des Gehäuses 1 erreicht, wobei mögliche Auslenkungen des Metallprodukt 1 zur Seite hin weniger wahrscheinlich bis gar nicht möglich sind. Damit kann wie vorstehend bereits erläutert verhindert werden, dass das Metallprodukt 1 unbeabsichtigt in Kontakt mit den Stirnseiten der Faltenbälge 23 gelangt und diese bzw. die dahinter angeordneten Komponenten der Messvorrichtung 16 beschädigt.

Fig. 14 zeigt eine weitere Variante in Bezug auf die Führungsrollen 28 der Schutz-Gleiteinrichtung in Verbindung mit einer Form des Gehäuses 11 gemäß Fig. 2. Bei der Ausführungsform von Fig. 14 können die Sendeeinheit 17 und die Empfangseinheit 18 in den Öffnungen 13, 14, die in gegenüberliegenden Seiten des Gehäuses 11 ausgebildet sind, nach dem Funktionsmechanismus gemäß Fig. 5 angeordnet sein.

Bei der Variante gemäß Fig. 14 können zwei Führungsrollen 28z in Richtung einer gegenüberliegenden Wandung des Gehäuses 11 vorzugsweise translatorisch bewegt werden, um dadurch in Kontakt mit dem Metallprodukt 1 zu gelangen und ggf. eine Druckkraft auf das Metallprodukt 1 auszuüben. In der Darstellung von Fig. 14 sind diese beweglichen Führungsrollen 28z auf der rechten Seite des Metallprodukts 1 angeordnet. Diese bewegbaren Führungsrollen 28z haben zueinander einen geringeren Abstand als zwei weitere Führungsrollen 28, die auf der entgegengesetzten Seite des Metallprodukts (in Fig. 14 auf der linken Seite) angeordnet sind. Falls nun die beweglichen Führungsrollen 28z, in Bezug auf die Zeichenebene Fig. 14, von rechts nach links bewegt werden, so wird damit erreicht, dass das bandförmige Metallprodukt 1 zwischen einerseits den beweglichen Führungsrollen 28z und andererseits den auf der anderen Seite des Metallprodukts 1 angeordneten beiden Führungsrollen 28 innerhalb des Innenraums 11i des Gehäuses 11 definiert geführt wird, so dass keine Gefahr einer Auslenkung des Metallprodukts 1 zur Seite hin mit einem nicht gewünschten Kontakt mit dem Faltenbalg 23 besteht. Des Weiteren führt das gezielte Anstellen der beweglichen Führungswalzen 28z gegen das bandförmige Metallprodukt 1 dazu, dass auf das Metallprodukt 1 eine Druckkraft ausgeübt wird, die sodann in einer Längserstreckung des Metallprodukts 1 eine Zugkraft hervorruft. In dieser Weise kann ein mögliches "Schlackern" des bandförmigen Metallprodukts 1 verhindert werden, um dadurch auch eine mögliche Beschädigung der Faltenbälge 23 bzw. der dahinter angeordneten Komponenten der Messvorrichtung 16 auszuschließen.

Mit Hilfe der Schutz-Gleiteinrichtung gemäß Fig. 14, bei der wie erläutert zwei bewegbare und drehbar gelagerten Führungsrollen 28z gegen das bandförmige Metallprodukt 1 angestellt werden, wird erreicht, dass das Metallprodukt 1 innerhalb des Innenraums 11i des Gehäuses zumindest im Bereich angrenzend zu den Öffnungen 13, 14 gezielt geführt wird. Entsprechend kann dann eine Stirnseite eines Faltenbalgs 23, welcher der Sendeeinheit 17 oder der Empfangseinheit 18 zugeordnet ist, und somit auch die zugehörige Komponente 17, 18 der Messvorrichtung 16 noch näher an dem Metallprodukt 1 angeordnet sein, beispielsweise mit einem Abstand von weniger als 10 mm, da in Folge der gezielten Führung des Metallprodukts 1 keine Gefahr eines seitlichen "Schlackerns" und somit einer Schädigung der Faltenbälge 23 und der dahinter angeordneten Komponenten 17, 18 der Messvorrichtung 16 besteht.

In Bezug auf die Fig. 14 darf ergänzend darauf hingewiesen werden, dass hier zwecks einer vereinfachten Darstellung, und wegen der vorliegenden Achsensymmetrie des Gehäuses 11 in der hier gezeigten vertikalen Richtung, ein Faltenbalg 23 lediglich an einer Seite des Gehäuses 11 und ohne eine hiervon umschlossene Komponente 17, 18 der Messvorrichtung 16 gezeigt ist. Des Weiteren ist ersichtlich, dass in der Fig. 14 ein Faltenbalg 23 derart in den Innenraum 11i des Gehäuses 11 hinein bewegt ist, dass seine dem Innenraum 11i zugewandte Stirnseite mit dem darin integrierten Fenster unmittelbar angrenzend an das Metallprodukt 1 positioniert ist. Zu erkennen ist auch, dass ein Abstand zwischen den beiden beweglichen Führungsrollen 28z derart groß gewählt ist, dass zwischen diesen Führungsrollen 28z der Faltenbalg 23 Platz hat, wenn er in direkter Nähe zum Metallprodukt 1 positioniert ist.

Fig. 15 zeigt eine Ausführungsform der Vorrichtung 10 in einer vereinfachten Seitenansicht, bei der die Komponenten der Messvorrichtung 16, d.h. die Sendeeinheit 17 und die Empfangseinheit 18, angrenzend zu den beiden Öffnungen 13, 14 in gleicher Weise angeordnet sein können wie bei dem Funktionsmechanismus von Fig. 5.

Aus der Fig. 15 ist ersichtlich, dass die Vorrichtung 10 auch eine Halteeinrichtung H umfasst, an der die Sendeeinheit 17 und die Empfangseinheit 18 gemeinsam angebracht sind. Die Halteeinrichtung H bildet somit eine Rahmeneinrichtung, die beispielsweise und gemäß der Darstellung von Fig. 15 C-förmig ausgebildet ist. Mit dieser Halteeinrichtung H können die Sendeeinheit 17 und die Empfangseinheit 18 in einer longitudinalen Richtung L parallel zu einer Oberfläche des bandförmigen Metallprodukts 1 beweglich verstellt werden können. Konkret bedeutet dies, dass die Sendeeinheit 17 und die Empfangseinheit 18, wenn sie zuvor in der transversalen Richtung ausreichend weit aus dem Innenraum 11i des Gehäuses 11 heraus bewegt worden sind (vgl. Fig. 6), dann anschließend mittels der Halteeinrichtung H in der longitudinalen Richtung L von dem Gehäuse 11 entfernt, d.h. weit weg bewegt und somit "aus der Linie gefahren" werden können.

Wenn die Sendeeinheit 17 und Empfangseinheit 18 wie soeben erläutert mittels der Halteeinrichtung H durch eine Bewegung in der longitudinalen Richtung L ausreichend weit von dem Gehäuse 11 entfernt worden sind, ist es möglich, die Messvorrichtung 16 mit diesen Komponenten 17, 18 für andere Messungen einzusetzen, beispielsweise zum Vermessen von kalibrierten bzw. geeichten Probekörpern zum Zwecke einer Kalibrierung der Komponenten 17, 18 der Vorrichtung 10. Ergänzend oder alternativ kann für die vorliegende Erfindung vorgesehen sein, für die Komponenten 17, 18 der Vorrichtung 10 Wartungs- und/oder Reparaturarbeiten durchzuführen, nachdem die Sendeeinheit 17 und die Empfangseinheit 18 wie erläutert durch eine Bewegung in longitudinaler Richtung mittels der Halteeinrichtung H von dem Gehäuse 11 entfernt worden sind.

In den Fig. 9 bis 12 sind weitere Ausführungsformen für die erfindungsgemäße Vorrichtung 10 gezeigt, nämlich jeweils in einer Längsschnittansicht durch das Gehäuse 11. Die Gemeinsamkeit für diese Ausführungsformen besteht darin, dass hierfür ein Gehäusetyp gemäß Fig. 2 zum Einsatz kommen, bei dem das Gehäuse 11 im Bereich der Öffnungen 13, 14 eine Engstelle 19 aufweist. Des Weiteren ist hierin die transversale Richtung, in der die Sendeeinheit 17 und die Empfangseinheit 18 mittels einer hiermit jeweils verbundenen Verstelleinrichtung 15a relativ zum Gehäuse 11 bewegbar sind, in gleicher Weise wie bei der Fig. 5 jeweils durch einen mit "T" bezeichneten Pfeil angedeutet.

Bei der Ausführungsform nach der Fig. 9 können die Sendeeinheit 17 und die Empfangseinheit 18 nach dem gleichen Funktionsmechanismus wie bei der Fig. 5 im Bereich der in der Wandung 12 des Gehäuses 11 ausgebildeten Öffnungen 13, 14 beweglich angebracht sein, wobei eine Abdichtung des Innenraums 11i des Gehäuses unter Verwendung der Faltenbälge 23 erreicht wird.

Die Darstellung von Fig. 9 verdeutlicht des Weiteren, dass sich die beiden Öffnungen 13, 14 jeweils durch eine Verschlusseinrichtung 26, hier jeweils nur durch eine gestrichelte Linie symbolisiert, verschlossen werden können, wenn die Komponenten 17, 18 der Messvorrichtung 16 ausreichend weit aus dem Innenraum 11 des Gehäuses heraus bewegt sind. Dies kann in gleicher Weise wie bei der Fig. 6 erfolgen, wo eine solche Verschlusseinrichtung 26 gezeigt und bereits erläutert worden ist.

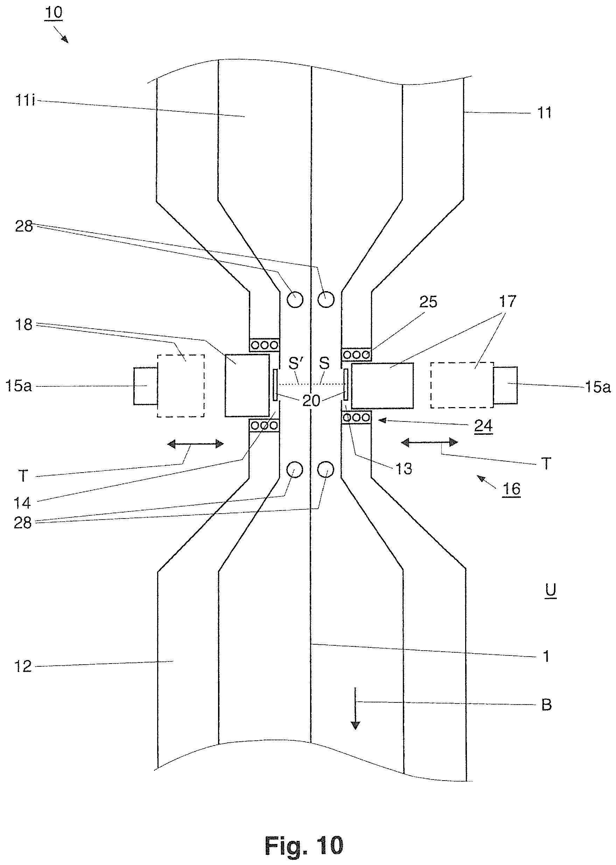

Bei der Ausführungsform von Fig. 10 kommen zur Abdichtung des Innenraums 11i keine Faltenbälge zum Einsatz; Dies deshalb nicht, weil die Fenster 20 direkt an dem Gehäuse 11 angrenzend zur ersten Öffnung 13 und zur zweiten Öffnung 14 befestigt sind und insoweit eine Abdichtung des Innenraums 11i gewährleistet ist. Des Weiteren verdeutlicht die Darstellung von Fig. 10, dass in dem Gehäuse 11i angrenzend zu den beiden Öffnungen 13, 14 jeweils Kühlkanäle 25 ausgebildet sind, um das Gehäuse 11 unmittelbar angrenzend an die daran angebrachten Fenster 20 zu kühlen.

Bei den Ausführungsformen gemäß Fig. 9 und Fig. 10 sind die Sendeeinheit 17 und die Empfangseinheit 18 durch strichlierte Linien jeweils in einer Position gezeigt, in der sie vollständig sowohl aus dem Innenraum 11i des Gehäuses 12 als auch aus den aus den ihnen zugeordneten Öffnungen 13, 14 heraus bewegt sind - ausgehend von dieser Position können dann diese Komponenten der Messvorrichtung 16 mittels der Halteeinrichtung H (vgl. Fig. 15), die zur Vereinfachung in den Fig. 9-12 nicht gezeigt ist, in der erläuterten Weise in longitudinaler Richtung L von dem Gehäuse 11 weg bewegt werden.

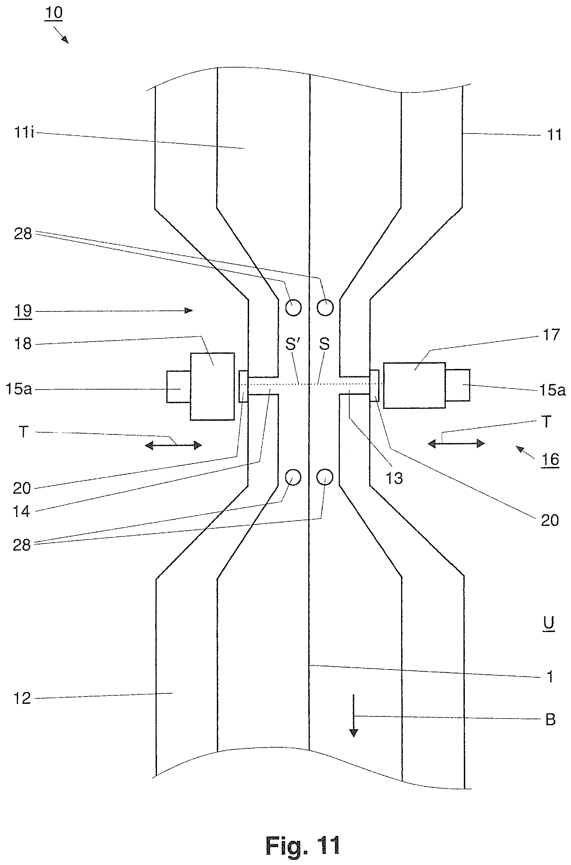

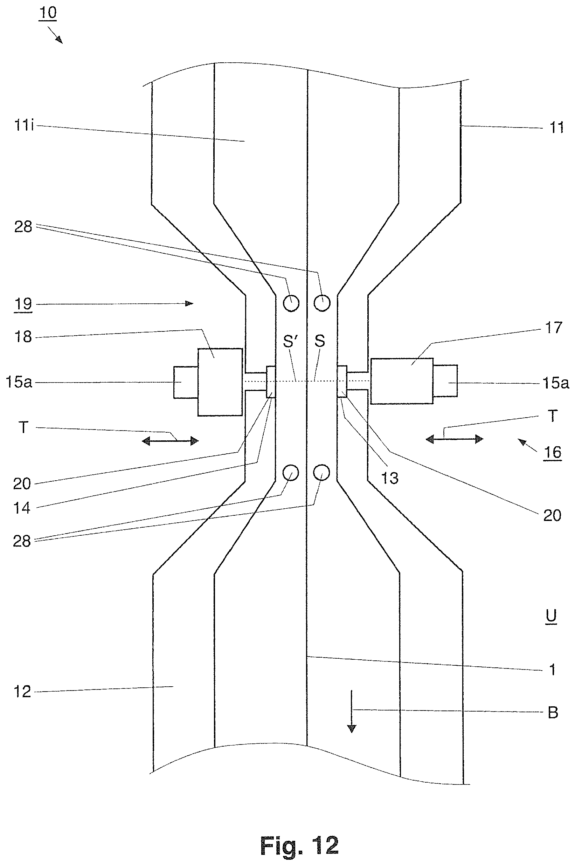

Bei den Ausführungsformen nach Fig. 11 und Fig. 12 sind die Fenster 20, in gleicher Weise wie bei der Ausführungsform von Fig. 10, direkt an dem Gehäuse 11 und angrenzend zu den Öffnungen 13, 14 angebracht. Insoweit bedarf es auch bei diesen Ausführungsformen nicht des Einsatzes eines Faltenbalgs, um eine Abdichtung des Innenraums 11i gegenüber der Umgebung U zu erreichen.

Allen der vorstehend genannten Ausführungsformen gemäß der Fig. 1-15 ist gemeinsam, dass dabei die Komponenten der Messvorrichtung 16, d.h. Sendeeinheit 17 und Empfangseinheit 18 auf jeweils entgegengesetzten Seiten des Metallprodukts 1 angeordnet sind. Dies hat zur Folge, dass bei diesen Ausführungsformen der Erfindung die von der Sendeeinheit 18 erzeugten Wellen, beispielsweise einer elektromagnetischen Strahlung, vorzugsweise Röntgenstrahlung, jeweils durch das Metallprodukt 1 hindurchtreten, was in den genannten Figuren jeweils mit einer mit S bezeichneten punktierten Linie symbolisiert ist. Das verbleibende und/oder resultierende Wellenmuster, in den genannten Zeichnungen jeweils mit einer mit S' bezeichneten punktierten Linie symbolisiert, wird dann auf der entgegengesetzten Seite des Metallprodukts 1 von der Empfangseinheit 18 empfangen.

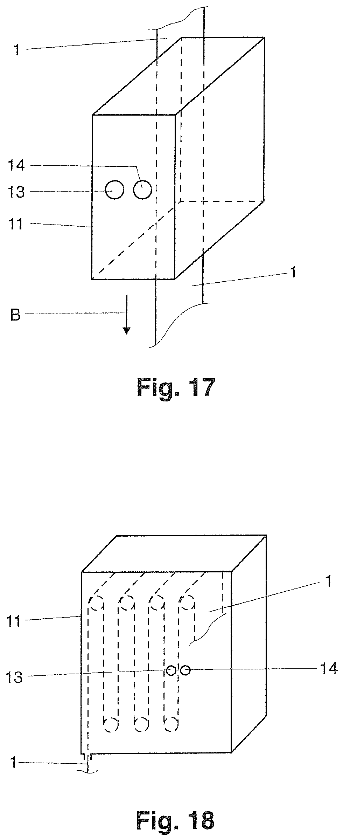

In den Fig. 17 bis 25 sind weitere erfindungsgemäße Ausführungsformen gezeigt. Hierbei ist das Dichtkonzept für den Innenraum 11i des Gehäuses 11 im Zusammenhang mit einer Beweglichkeit der Komponenten 17, 18 der Messvorrichtung 16 relativ zum Gehäuse 11 ebenfalls unter Verwendung von Faltenbälgen 23 mit darin integrierten Fenstern 20 realisiert, wobei diese Faltenbälge 23 - in gleicher Weise wie bereits zur Fig. 5 erläutert - an den Rändern der Öffnungen 13, 14 befestigt sind. Alle Faltenbälge 23 der Ausführungsformen gemäß Fig. 17-25 sind mit Verstellmitteln verbunden, mit denen ein Bewegen dieser Faltenbälge 23 hinein in den Innenraum 11i des Gehäuses 11 und auch in die Gegenrichtung, d.h. heraus aus dem Gehäuse 11 möglich ist. Zur Vereinfachung sind diese Verstellmittel in den Fig. 17-25 nicht gezeigt. Beispielsweise ist auch eine Bewegung dieser Faltenbälge 23 unabhängig voneinander und insbesondere für den Fall möglich, dass die zugeordneten Komponenten 17, 18 der Messvorrichtung 16 sich jeweils außerhalb des Gehäuses 11 der Vorrichtung 10 befinden.

Bei den Ausführungsformen nach den Fig. 17 und Fig. 18 umfasst die Vorrichtung 10 jeweils ein Gehäuse 11, bei dem die beiden Öffnungen 13, 14 nebeneinander in einer Wandung 12 ausgebildet sind, die fluchtend zur Oberfläche des bandförmigen Metallprodukts 1 angeordnet ist. Hierbei ist in den Perspektivansichten gemäß der Fig. 17 und Fig. 18 sowohl die bandförmige Ausbildung des Metallprodukts 1 zu erkennen als auch die Bewegungsrichtung B gezeigt, in der das bandförmige Metallprodukt 1 durch das Gehäuse 11 dieser Ausführungsformen hindurchbewegt wird.

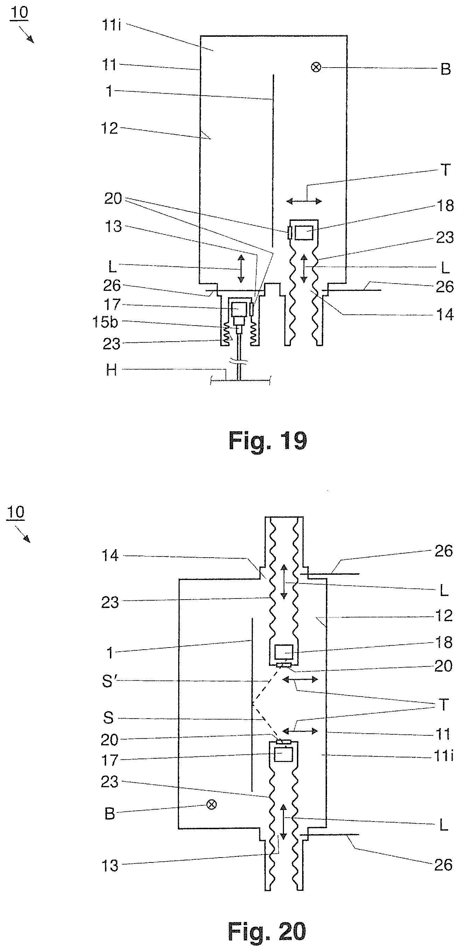

Die Darstellung von Fig. 19 zeigt einen Querschnitt des Gehäuses 11 von Fig. 17. Wie soeben erläutert, sind hierbei die erste Öffnung 13 und die zweite Öffnung 14 in einer gleichen Wandung 12 des Gehäuses 11 nebeneinander ausgebildet, wobei die Sendeeinheit 17 der ersten Öffnung 13 und die Empfangseinheit 18 der zweiten Öffnung zugeordnet sind.

In gleicher Weise wie bei den vorherigen Ausführungsformen sind bei der Ausführungsform von Fig. 19 die Sendeeinheit 17 und die Empfangseinheit 18 jeweils mit Verstelleinrichtungen wirkverbunden, die hier mit "15b" bezeichnet sind und wie bereits erläutert unabhängig voneinander aktuiert werden können. Zwecks einer vereinfachten Darstellung ist in der Fig. 19 die Verstelleinrichtung für die Empfangseinheit 18 nicht gezeigt.

Im Hinblick auf die bandförmige Ausgestaltung des Metallprodukts 1 werden die Sendeeinheit 17 und/oder die Empfangseinheit 18 bei einer Aktuierung einer damit verbundenen Verstelleinrichtung 15b in longitudinaler Richtung L bewegt, d.h. parallel zu einer Oberfläche des bandförmigen Metallprodukts 1, und können dadurch entweder in den Innenraum 11i des Gehäuses 11 hinein bewegt oder aus dem Gehäuse 11 heraus bewegt werden.

In Bezug auf die Darstellung von Fig. 19 wird auf folgende weitere Aspekte hingewiesen:
- Die Sendeeinheit 17 und die Empfangseinheit 18 sind beispielhaft und im Vergleich zueinander in verschiedenen Betriebsstellungen gezeigt: Die Sendeeinheit 17 befindet sich angrenzend zur ersten Öffnung 13 und außerhalb des Innenraums 11i des Gehäuses 11, wohingegen die Empfangseinheit 18 in longitudinaler Richtung L in den Innenraum 11i des Gehäuses 11 hinein bewegt ist.
- In seitlichen Bereichen der Faltenbälge 23, die der Sendeeinheit 17 und der Empfangseinheit 18 jeweils zugeordnet sind und diese umschließen, sind jeweils Fenster 20 integriert, in gleicher Weise wie bei der Fig. 5 bereits erläutert.
- Angrenzend zu den beiden Öffnungen 13, 14 - in gleicher Weise wie bereits zur Fig. 6 erläutert - sind an dem Gehäuse 11 Verschlusseinrichtungen 26 angebracht. Falls die Komponenten 17, 18 der Messvorrichtung 16 außerhalb des Innenraums 11i des Gehäuses 11 angeordnet sind, kann eine Öffnung 13, 14 durch eine zugeordnete Verschlusseinrichtung 26 verschlossen werden. In Fig. 19 ist dies für die erste Öffnung 13 der Fall.
- Falls die Verschlusseinrichtung 26, welche der ersten Öffnung 13 zugeordnet ist, in ihre Offenstellung überführt wird, so wie es in der Fig. 19 für die der zweiten Öffnung 14 zugeordneten Verschlusseinrichtung 26 der Fall ist, kann die Sendeeinheit 17 durch eine Aktuierung der Verstelleinrichtung 15b in longitudinaler Richtung L in den Innenraum 11i des Gehäuses 11 hinein bewegt werden, beispielsweise in die Position, in der auch die Empfangseinheit 18 gezeigt ist. Diesbezüglich versteht sich, dass dabei der Faltenbalg 23, welcher die Sendeeinheit 17 umschließt, entweder synchron mit der Sendeeinheit 17 bewegt wird oder zuvor in das Gehäuse 11 hinein bewegt worden ist.
- Auch in Bezug auf die Empfangseinheit 18 und im Hinblick auf die in Fig. 19 gezeigt Position versteht sich, dass der zugeordnete Faltenbalg 23 entweder synchron mit der Empfangseinheit 18 oder zuvor bewegt worden ist.
- Falls auch die Sendeeinheit 17 durch ein Bewegen in longitudinaler Richtung L in die Position überführt worden ist, in der in Fig. 19 die Empfangseinheit 18 gezeigt ist, sind dann diese beiden Komponenten 17, 18 der Messvorrichtung 16 jeweils auf entgegengesetzten Seiten des bandförmigen Metallprodukts 1 angeordnet, wo die in den zugehörigen Faltenbälgen 23 integrierten Fenster 20 jeweils dem bandförmigen Metallprodukt 1 zugewandt sind. Entsprechend kann dann zumindest eine Eigenschaft des Metallprodukts 1 nach dem Transmissions- bzw. Durchstrahlprinzip ermittelt werden, in gleicher Weise wie bereits zur Fig. 5 erläutert.
- Nachdem die Sendeeinheit 17 und/oder die Empfangseinheit 18 in den Innenraum 11i des Gehäuses 11 hinein bewegt worden sind, so wie es der für die Empfangseinheit 18 gezeigten Position entspricht, ist es möglich, durch eine entsprechende Betätigung der Verstelleinrichtung 15b die Sendeeinheit 17 und/oder die Empfangseinheit 18 auch in transversaler Richtung T zu bewegen, um dadurch einen Abstand zum Metallprodukt 1 gezielt zu verändern und auf einen vorbestimmten Wert einzustellen.
- Die Verstelleinrichtungen 15b, die der Sendeeinheit 17 und der Empfangseinheit 18 zugeordnet sind, sind - in gleicher Weise wie die Verstelleinrichtung 15a bei der Ausführungsform von Fig. 15 - an einer Halteeinrichtung L befestigt. Dies ist in Fig. 19 zur Vereinfachung nur für die mit der Sendeeinheit 17 verbundenen Verstelleinrichtung 15a gezeigt. Falls Sendeeinheit 17 und Empfangseinheit 18 sich jeweils außerhalb des Gehäuses 11 befinden und weit genug von den Öffnungen 13, 14 des Gehäuses 11 beabstandet sind, kann mittels einer Aktuierung der Halteeinrichtung H erreicht werden, dass Sendeeinheit 17 und Empfangseinheit 18 beispielsweise in transversaler Richtung T von dem Gehäuse 11 entfernt werden, um an anderer Stelle beispielsweise eine Kalibrierungsmessung und/oder Wartungs- bzw. Reparaturarbeiten durchzuführen.
- Und schließlich: Die Bewegungsrichtung B für das bandförmige Metallprodukt 1 erstreckt sich bei der Darstellung von Fig. 19 hinein in die Zeichenebene.

Die Ausführungsform von Fig. 20 umfasst ein Gehäuse 11 ähnlich zu jenem von Fig. 17, mit dem Unterschied, dass hierbei die erste Öffnung 13 und die zweite Öffnung 14 nicht in der gleichen Wandung 12, sondern in einander gegenüberliegenden Wandungen 12 des Gehäuses ausgebildet sind. Mit der Maßgabe, dass auch bei der Ausführungsform von Fig. 20 die Sendeeinheit 17 der ersten Öffnung 13 und die Empfangseinheit 18 der zweiten Öffnung 14 zugeordnet sind, entspricht der Funktionsmechanismus, mit dem hierbei ein Bewegen der Komponenten 17, 18 der Messvorrichtung 16 in longitudinaler Richtung L in den Innenraum 11i des Gehäuses 11 oder aber in die Gegenrichtung, d.h. heraus aus dem Gehäuse 11, als auch ein Verschließen des Innenraums 11i mittels der Verschlusseinrichtungen 26 möglich ist, jenem von Fig. 19, so dass zur Vermeidung von Wiederholungen auf die Erläuterungen zur Fig. 19 verwiesen werden darf. Ergänzend wird angemerkt, dass bei der Darstellung von Fig. 20 zwecks einer vereinfachten Darstellung die Verstelleinrichtung 15b und die Halteeinrichtung L, die in gleicher Weise wie bei der Ausführungsform von Fig. 15 ausgebildet sein kann, nicht gezeigt sind.

Die Ausführungsform gemäß Fig. 20 zeichnet sich dadurch aus, dass die Sendeeinheit 17 und die Empfangseinheit 18, wenn sie gemäß der Darstellung von Fig. 20 in den Innenraum 11i des Gehäuses 11 hinein bewegt sind, dann auf der gleichen Seite des bandförmigen Metallprodukts 1 angeordnet sind. Entsprechend erfolgt ein Ermitteln von zumindest einer Eigenschaft des Metallprodukts 1 nach dem Reflexionsprinzip, indem die Wellen einer von der Sendeeinheit 17 erzeugten elektromagnetischen Strahlung S durch das Fenster 20 des Faltenbalgs 23 hindurch auf das Metallprodukt 1 gerichtet werden, wobei das verbleibende und/oder resultierende Wellenmuster S' dann von der Empfangseinheit 18 empfangen wird. Im Übrigen entspricht bei der Ausführungsform von Fig. 20 die Bewegungsrichtung B für das bandförmige Metallprodukt 1 jener von Fig. 19 und erstreckt sich hinein in die Zeichenebene.

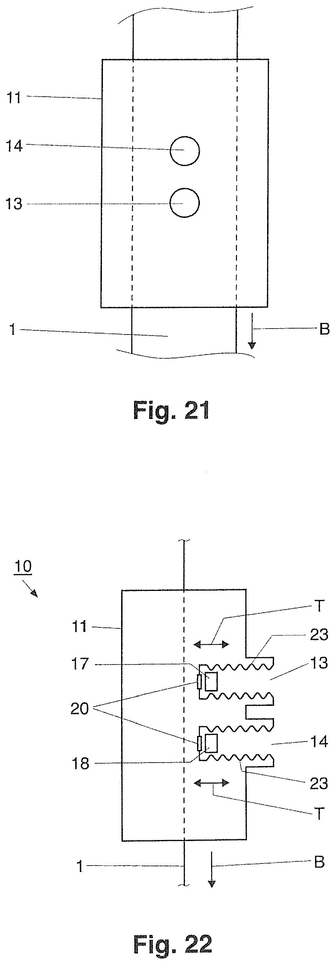

Die Ausführungsform von Fig. 21 umfasst ein Gehäuse 11, bei dem die ersten und zweiten Öffnungen 13, 14 in der gleichen Wandung 12 ausgebildet sind. Dieses Gehäuse 11 ist derart beschaffen, dass ein bandförmiges Metallprodukt 1 vertikal hierdurch hindurchgeführt wird, wie es für die Seitenansicht von Fig. 21 durch den Pfeil B für die Bewegungsrichtung des Metallprodukts 1 symbolisiert ist.

Fig. 22 zeigt das Gehäuse 11 von Fig. 21 in einer Längsschnittansicht.

Bei der Ausführungsform gemäß Fig. 21 und Fig. 22 wird ebenfalls das Prinzip verfolgt, dass die Sendeeinheit 17 der ersten Öffnung 13 und die Empfangseinheit 18 der zweiten Öffnung 14 zugeordnet sind. Der Funktionsmechanismus für ein Bewegen dieser Komponenten 17, 18 der Messvorrichtung 16 im Bereich der Öffnungen 13, 14 und auch einem Bewegen der Faltenbälge 23 entspricht in gleicher Weise der Ausführungsform von Fig. 19, sodass zur Vermeidung von Wiederholungen auf die Erläuterungen zur Fig. 19 verwiesen wird.

Die Darstellung von Fig. 22 zeigt die Sendeeinheit 17 und die Empfangseinheit 18 jeweils in einer Position, in der sie in den Innenraum 11i des Gehäuses 11 hinein bewegt sind. Die Sendeeinheit 17 und die Empfangseinheit 18 gelangen in diese Position, indem sie durch eine Aktuierung der jeweils zugeordneten (und hier nicht gezeigten) Verstelleinrichtungen jeweils in transversaler Richtung T bewegt werden. Fig. 22 verdeutlicht, dass die Sendeeinheit 17 und die Empfangseinheit 18 in diesen Positionen jeweils auf der gleichen Seite des bandförmigen Metallprodukts 1 angeordnet sind. Entsprechend erfolgt im Betrieb der Vorrichtung 1 und der zugehörigen Messvorrichtung 16 das Ermitteln von zumindest einer Eigenschaft des bandförmigen Metallprodukts 1 nach dem Reflexionsprinzip, in gleicher Weise wie auch bei der Ausführungsform gemäß Fig. 20.

Ergänzend wird zur Fig. 22 darauf hingewiesen, dass hierbei für die Sendeeinheit 17 und die Empfangseinheit 18 eine gemeinsame Verstelleinrichtung 15b vorgesehen sein kann, mit der dann eine Bewegung dieser Komponenten 17, 18 der Messvorrichtung 16 in der transversalen Richtung realisiert wird.

Die Ausführungsform von Fig. 23 entspricht einer Variante zur Ausführungsform gemäß der Fig. 21 bzw. Fig. 22, mit der Modifikation, dass hierbei die beiden Öffnungen 13, 14 nicht separat voneinander und übereinander in der gleichen Wandung 12 des Gehäuses 11 ausgebildet sind, sondern stattdessen in einer gemeinsamen Öffnung G zusammengefasst sind. Dies ist in der Darstellung von Fig. 23 ersichtlich, die eine Querschnittsansicht durch das Gehäuse 11 zeigt.

In der Darstellung von Fig. 23 sind die Sendeeinheit 17 und die Empfangseinheit 18 jeweils in einer Position gezeigt, wenn sie in transversaler Richtung T in den Innenraum 11i des Gehäuses hinein bewegt sind. Hierbei ist ersichtlich, dass die Komponenten 17, 18 der Messvorrichtung 16, in gleicher Weise wie bei der Fig. 20, auf der gleichen Seite des bandförmigen Metallprodukts 1 angeordnet sind, so dass im Betrieb der Messvorrichtung 16 zumindest einer Eigenschaft des bandförmigen Metallprodukts nach dem Reflexionsprinzip ermittelt wird.

In Anbetracht dessen, dass bei der Ausführungsform von Fig. 23 die Komponenten 17, 18 der Messvorrichtung 16 durch die gemeinsame Öffnung G des Gehäuses 11 hindurch bewegt werden können, kommt für eine Realisierung dieser Bewegung zweckmäßigerweise auch nur eine (in Fig. 23 nicht gezeigte) Verstelleinrichtung zum Einsatz, mit der die Sendeeinheit 17 und die Empfangseinheit 18 wirkverbunden sind.

Bei der Ausführungsform von Fig. 23 ist vorzugsweise ein gemeinsamer Faltenbalg 23 für Sendeeinheit 17 und Empfangseinheit 18 vorgesehen, wobei dieser Faltenbalg 23 an den Rändern der Öffnung G befestigt ist und sich mittels einer zugeordneten Verstelleinheit relativ zur Öffnung G des Gehäuses 11 und in transversaler Richtung T bewegen lässt. An einer Stirnseite dieses gemeinsamen Faltenbalgs 23, die dem bandförmigen Metallprodukt 1 zugewandt ist, ist in gleicher Weise wie bei der Fig. 22 ein (nicht gezeigtes) Fenster integriert, welches wie erläutert in Bezug auf die Wellen der elektromagnetischen Strahlung durchlässig ist, um das Vermessen des Metallprodukts 1 nach dem Reflexionsprinzip zu ermöglichen.

Die Ausführungsform gemäß Fig. 24 stellt eine Variante zur Ausführungsform von Fig. 19 dar, mit der Modifikation, dass hierbei die erste und zweite Öffnung derart in der Wandung 12 des Gehäuses 11 ausgebildet sind, dass sie zu einer gemeinsamen Öffnung G zusammengefasst sind. Analog zur Fig. 23 sind bei der Ausführungsform von Fig. 24 zweckmäßigerweise nur ein Faltenbalg 23, der an den Rändern der gemeinsamen Öffnung G befestigt ist, und auch nur eine Verstelleinrichtung vorgesehen, mit der Sendeeinheit 17 und Empfangseinheit 18 jeweils wirkverbunden sind und sich bei einer Aktuierung dieser Verstelleinrichtung in den Innenraum 11i des Gehäuses 11 in longitudinaler Richtung L hinein bewegen lassen

In gleicher Weise wie die Fig. 19 zeigt die Darstellung von Fig. 24 ebenfalls einen Querschnitt durch das Gehäuse 11 und die Komponenten 17, 18 in einer jeweiligen Betriebsposition, wenn sie auf jeweils entgegengesetzten Seiten des bandförmigen Metallprodukts 1 angeordnet sind. Ferner verdeutlicht Fig. 24, dass in seitlichen Bereichen des Faltenbalgs 23 zwei Fenster 20 integriert sind, die, in gleicher Weise wie bei der Fig. 19, der Sendeeinheit 17 und der Empfangseinheit zugeordnet sind. Dies ermöglicht ein Ermitteln von zumindest einer Eigenschaft des bandförmigen Metallprodukts 1 nach dem Transmissions- bzw. Durchstrahlprinzip.

Die Ausführungsformen gemäß Fig. 23 und Fig. 24 sind in gleicher Weise wie beispielsweise die Ausführungsform von Fig. 19 mit Verschlusseinrichtungen 26 ausgestattet, die jeweils im Bereich der gemeinsamen Öffnung G am Gehäuse 11 angebracht ist. Falls die Sendeeinheit 17 und die Empfangseinheit 18 sich außerhalb des Gehäuses 11 befinden, kann der Innenraum 11i des Gehäuses mittels dieser Verschlusseinrichtung 26 verschlossen werden, so wie es vorstehend beispielsweise zur Fig. 19 oder zur Fig. 6 bereits erläutert worden ist.

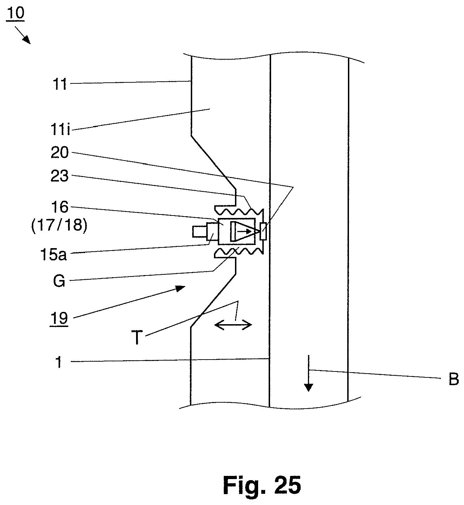

Schließlich zeigt Fig. 25 eine weitere Ausführungsform der erfindungsgemäßen Vorrichtung 10, nämlich hier in einer Längsschnittansicht durch das Gehäuse 11 dieser Vorrichtung 10. In Bezug auf diese Ausführungsform ist hervorzuheben, dass hierbei die Sendeeinheit 17 und die Empfangseinheit 18 der Messvorrichtung 16 zu einem integrierten Messkopf zusammengefasst sind.

Bei der Ausführungsform gemäß Fig. 25 hat die genannte Integration von Sendeeinheit 17 und Empfangseinheit 18 in einen gemeinsamen Messkopf zur Folge, dass diese Komponenten der Messvorrichtung 16 auf der gleichen Seite des bandförmigen Materials 1 angeordnet sind. Entsprechend erfolgt das Ermitteln von zumindest einer Eigenschaft des bandförmigen Metallprodukts hier nach dem Reflexionsprinzip.

Die Ausführungsform gemäß Fig. 25 eignet sich insbesondere für den Einsatz von Laserstrahlung, die von der Sendeeinheit 17 erzeugt und auf die Oberfläche des Metallprodukts 1 gerichtet wird, wobei der reflektierte Teil dieser Laserstrahlung dann von der Empfangseinheit 18 empfangen wird. Gleiches gilt auch für die Ausführungsformen gemäß Fig. 22 und Fig. 23.

In Bezug auf die Ausführungsform von Fig. 25 wird ergänzend darauf hingewiesen, dass es sich bei dem hier gezeigten Messkopf alternativ auch um einen IMPOC-Messkopf handeln kann. Einzelheiten zum IMPOC-Messverfahren sind vorstehend bereits erläutert worden.

Für die Ausführungsformen nach den Fig. 20-25 darf ergänzend darauf hingewiesen werden, dass hierbei auch eine (nicht gezeigte) Halteeinrichtung vorgesehen sein kann, an der die Sendeeinheit 17 und die Empfangseinheit 18 angebracht sind, analog zur Ausführungsform von Fig. 15 oder von Fig. 19. Mit einer solchen Halteeinrichtung ist es möglich, die Sendeeinheit 17 und die Empfangseinheit 18 von dem Gehäuse 11 zu entfernen bzw. weg zu bewegen, nachdem diese Komponenten 17, 18 der Messvorrichtung 16 aus dem Innenraum 11i heraus bewegt worden sind und sich entsprechend außerhalb des Gehäuses 11 befinden. Hinsichtlich der Durchführung von möglichen Kalibrierungsmessungen darf zur Vermeidung von Wiederholungen auf die Erläuterungen zur Fig. 15 verwiesen werden.

In Bezug auf alle der vorstehend genannten Ausführungsformen der erfindungsgemäßen Vorrichtung 10 versteht sich, dass hierbei auch die Kühlmaßnahmen und/oder die Spülgaseinrichtung zum Einsatz kommen können, die im Zusammenhang mit Fig. 7 und Fig. 8 gezeigt und erläutert worden sind. Dass damit beispielsweise eine Anpassung der Wandungen 12 des Gehäuses und/oder der Faltenbälge 23 und oder der Fenster 20, die jeweils in die Faltenbälge 23 integriert sein können, einhergeht, versteht sich von selbst und bedarf keiner weiteren Erläuterung.

In gleicher Weise versteht sich, dass bei allen der vorstehend genannten Ausführungsformen der erfindungsgemäßen Vorrichtung 10 die hierbei eingesetzten Verstelleinrichtungen 15a, 15b, mit denen ein Bewegen für die Komponenten 17, 18 der Messvorrichtung 16 realisiert wird, jeweils teleskopartig ausgebildet sein können, so wie es beispielsweise zur Fig. 5 erläutert worden ist.

In Bezug auf alle der vorstehend genannten Ausführungsformen der erfindungsgemäßen Vorrichtung 10 wird darauf hingewiesen, dass hierbei die Sendeeinheit 17 derart ausgebildet sein kann, dass mit ihr eine Laserstrahlung S erzeugt wird, die auf das Metallprodukt 1 gerichtet wird und in dessen Material Ultraschall erzeugt. Ein solches lokales Ultraschall-Feld in dem Material des Metallprodukts 1 kann ebenfalls mittels Laserstrahlung detektiert werden. Dies bedeutet, dass zu diesem Zweck dann auch die Empfangseinheit 18 dazu ausgebildet ist, Laserstrahlung zu erzeugen, die zwecks einer Messung des in dem in dem Material des Metallprodukts 1 erzeugten Ultraschalls auf das Metallprodukt gerichtet wird.

Die vorstehend genannten Ausführungsformen einer erfindungsgemäßen Vorrichtung 10 und ein hiermit durchgeführtes Verfahren gemäß der vorliegenden Erfindung können folgende weitere Merkmale aufweisen:
- Die Wandung 12 des Gehäuses 11 kann zumindest in Teilbereichen oder überall doppelwandig ausgebildet sein. Dies verbessert eine Isolation des Innenraums 11i des Gehäuses 11 gegenüber der Umgebung unvermindert gleichzeitig das Risiko einer möglichen Leckage bzw. das Entweichen von Gasen heraus aus dem Innenraum 11i. In gleicher Weise kann hierdurch verbessert verhindert werden, dass Luft aus der Umgebung in den Innenraum 11i des Gehäuses hineingelangt.
- Es ist eine Signalvorrichtung vorgesehen, mit der eine Schädigung des bandförmigen Metallprodukts 1, beispielsweise ein Reißen ("*strip break*"), bei seinem Transport durch das Gehäuse 11 hindurch erkannt werden kann. Für diesen Fall werden die Sendeeinheit 17 und die Empfangseinheit 18, ggf. in Verbindung mit den jeweils zugeordneten Faltenbälgen 23, unverzüglich aus dem Innenraum 11i des Gehäuses 11 herausbewegt, um eine Schädigung dieser Komponenten 17, 18 der Messvorrichtung 16 zu verhindern. Nachdem sich die Sendeeinheit 17 und die Empfangseinheit 18 außerhalb des Gehäuses 1 befinden, kann dann der Innenraum 11i mit seinen Öffnungen 13, 14, G mittels der Verschlusseinrichtung 26 verschlossen werden.

- Es ist eine Signaleinrichtung vorgesehen, mit der ein mögliches "Schlackern" des Metallprodukts 1, d.h. eine unzulässige Bewegung zur Seite hin, bei seinem Transport durch das Gehäuse 11 hindurch erkannt werden kann. Für diesen Fall kann die Signaleinrichtung eine Aktuierung der bewegbaren Führungsrollen 28z gemäß der Fig. 14 auslösen, die dann wie erläutert gegen das bandförmige Metallprodukt 1 wie bei Fig. 14 erläutert angestellt werden, um zumindest im Bereich der Fenster 13, 14 des Gehäuses 11 und der angrenzend hierzu beweglich geführten Komponenten 17, 18 der Messvorrichtung 16 eine genau definierte Führung des Metallprodukts 1 vorbei an der Sendeeinheit 17 und der Empfangseinheit 18 zu erreichen.
- Eine Kalibrierungsmessung für die Komponenten 17, 18 der Messvorrichtung kann auch dadurch erreicht werden, dass ein (nicht gezeigter) Probenkörper zwischen zwei verschiedenen Chargen eines Metallprodukts 1 befestigt wird und dann in gleicher Weise wie die Produktionschargen eines Metallprodukts durch das Gehäuse 11 hindurch transportiert wird. Beispielsweise kann ein solcher Probenkörper mit den Enden eines vorhergehenden Metallprodukts und eines später verwendeten Metallprodukts verschweißt werden, so dass der erfindungsgemäße Online-Messbetrieb keine nennenswerte Verzögerung erfährt. Jedenfalls wird dann ein solcher Probenkörper in gleicher Weise wie das normale Metallprodukt 1 bei dem Transport durch das Gehäuse 11 hindurch an der Sendeeinheit 17 und an der Empfangseinheit 18 vorbeigeführt, was dann eine solche Kalibrierungsmessung möglich macht.
- Es ist eine Detektionseinrichtung für den Bandverlauf des Metallprodukts 1 insbesondere angrenzend zu den Öffnungen 13, 14, G des Gehäuses 11 und der hierin beweglich geführten Komponenten 17, 18 der Messvorrichtung 16 vorgesehen. Gleichzeitig sind die Verstelleinrichtungen 15a, 15b jeweils Hochgeschwindigkeits-Stellzylinder ausgebildet, mit denen ein Bewegen der Sendeeinheit 17 und/oder der Empfangseinheit mit hoher Ansprech- bzw. Stellgeschwindigkeit möglich ist. In gleicher Weise können auch die Verstellmittel für die Faltenbälge 23 derart ausgebildet sein, dass mit ihnen ein Bewegen der damit verbundenen Faltenbälge 23 in sehr kurzer Zeit bzw. mit hoher Ansprechgeschwindigkeit möglich ist. Die besagte Detektionseinrichtung ist signaltechnisch mit den Verstelleinrichtungen bzw. Hochgeschwindigkeits-Stellzylindern und ebenso mit den Verstellmitteln für die Faltenbälge verbunden. In Folge dessen ist es möglich, dass die Sendeeinheit 17 und/oder Empfangseinheit 18 sehr schnell von dem Metallprodukt 1 wegbewegt werden, falls dieses eine außerplanmäßige Bewegung zur Seite hin, d.h. in Richtung der Komponenten 17, 18 der Messvorrichtung 16 erfahren sollte. Im Ergebnis kann damit eine Schädigung dieser Komponenten 17, 18 verhindert werden. Zweckmäßigerweise werden dann die jeweiligen Faltenbälge 23 synchron bzw. gleichzeitig mit den ihnen zugeordneten Komponenten 17, 18 der Messvorrichtung 16 von dem Metallprodukt wegbewegt. Für eine solche Ausführungsform der Erfindung ist es möglich, den Abstand zwischen einerseits der Sendeeinheit 17 und/oder der Empfangseinheit 18 und andererseits dem Metallprodukt auch auf Werte < 10 mm einzustellen.

### Bezugszeichenliste

- 1: Metallprodukt
- 10: Vorrichtung
- 11: Gehäuse
- 11i: Innenraum (des Gehäuse 11)
- 12: Wandung
- 13: erste Öffnung
- 14: zweite Öffnung
- 15a: Verstelleinrichtung
- 15b: Verstelleinrichtung
- 16: Messvorrichtung
- 17: Sendeeinheit (als Komponente der Messvorrichtung 16)
- 18: Empfangseinheit (als Komponente der Messvorrichtung 16)
- 19: Engstelle (des Gehäuses 11)
- 20: Abschirmung (z.B. in Form eines Fensters)
- 21: einzelne Schichten bzw. Lagen (einer Abschirmung bzw. eines Fensters 20)
- 22: Kavität
- 23: verformbare Dichteinrichtung (z.B. in Form eines Faltenbalgs)
- 24: Kühleinrichtung
- 25: Leitungsschlange oder Kühlkanal
- 26: Verschlusseinrichtung
- 27: Behälter (für Verschlusseinrichtung 26)
- 28: Schutz-Gleiteinrichtung (z.B. in Form einer drehbar gelagerten Führungsrolle)
- 30: Kühlleitung oder Kavität
- 32: Verstellmittel (für die verformbare Dichteinrichtung 23)
- 33: Stützstruktur (für die verformbare Dichteinrichtung 23)
- B: Bewegungsrichtung (für das Metallprodukt 1)
- F: Richtung, in der Spülgas auf das Fenster 20 gerichtet wird
- G: gemeinsame Öffnung (in der Wandung 12 des Gehäuses 11)
- H: Halteeinrichtung
- K: Kühlfluid
- L: longitudinale Bewegung (einer Komponente 17, 18 der Messvorrichtung 16)
- S: Signal, das von der Sendeeinheit 17 ausgesendet wird
- S': Signal, das von der Empfangseinheit 18 empfangen wird
- T: transversale Bewegung (einer Komponente 17, 18 der Messvorrichtung 16)
- U: Umgebung (des Gehäuses 11)

## Patentansprüche

1. Vorrichtung (10) zum berührungslosen Ermitteln von zumindest einer Eigenschaft eines Metallprodukts (1) während einer metallurgischen Herstellung des Metallprodukts (1), umfassend
zumindest eine Messvorrichtung (16) bestehend aus einer Sendeeinheit (17) und einer Empfangseinheit (18),
wobei zumindest von der Sendeeinheit (17) ein elektromagnetisches Feld erzeugt und auf das Metallprodukt (1) gerichtet wird und dadurch in dem Material des Metallprodukts (1) eine physikalische Wechselwirkung induziert wird, wobei ein verbleibender und/oder resultierender Teil dieser physikalischen Wechselwirkung von der Empfangseinheit (18) empfangen wird,
**dadurch gekennzeichnet, dass** die Vorrichtung (10)
ein Gehäuse (11) umfasst, durch welches das Metallprodukt (1) hindurch bewegbar ist, wobei das Gehäuse (11) Teil eines Ofens zum Wärmebehandeln und/oder Teil einer Anlage zum Beschichten des Metallprodukts (1) ist
wobei in der Wandung (12) des Gehäuses (11) eine erste Öffnung (13) und eine zweite Öffnung (14) ausgebildet sind, wobei die Sendeeinheit (17) der ersten Öffnung (13) zugeordnet ist, so dass das von der Sendeeinheit (17) erzeugte elektromagnetische Feld auf der Seite der ersten Öffnung (13) auf das Metallprodukt (1) auftrifft, wobei die Empfangseinheit (18) der zweiten Öffnung (14) zugeordnet ist, so dass der verbleibende und/oder resultierende Teil der in dem Material des Metallprodukts (1) induzierten physikalischen Wechselwirkung von der Empfangseinheit (18) auf der Seite der zweiten Öffnung (14) empfangen wird,
wobei das Gehäuse (11) im Bereich der ersten Öffnung (13) und/oder der zweiten Öffnung (14) eine Engstelle (19) aufweist,
wobei im Bereich der ersten Öffnung (13) und der zweiten Öffnung (14) jeweils eine Abschirmung (20) insbesondere in Form eines Fensters vorgesehen ist, die in Bezug auf die Wellen der elektromagnetischen Strahlung oder einer zugehörigen physikalischen Wirkung durchlässig ist, wobei die Abschirmung (20) derart beschaffen ist, dass damit Wärmestrahlung durch die Öffnungen (13, 14) hindurch aus dem Innenraum (11i) des Gehäuses (11) an dessen Umgebung (U), in der sich die Sendeeinheit (17) und die Empfangseinheit (18) befinden, vermindert ist
und
wobei außerhalb des Gehäuses (11) zumindest eine Verstelleinrichtung (15a, 15b) vorgesehen ist, mit der zumindest eine Komponente (17, 18) der Messvorrichtung (16) bestehend aus der Sendeeinheit (17) und/oder der Empfangseinheit (18) relativ zu der Wandung (12) des Gehäuses (11) und im Bereich einer Öffnung (13, 14) des Gehäuses (11) oder angrenzend hierzu bewegbar ist, um dadurch für diese Komponente (17, 18) einen vorbestimmten Abstand zu dem innerhalb des Gehäuses (11) bewegten Metallprodukt (1) einzustellen oder gezielt zu verändern.

2. Vorrichtung (10) nach Anspruch 1, **dadurch gekennzeichnet, dass** das Metallprodukt (1) aus einem bandförmigen Material gebildet ist, wobei die Verstelleinrichtung (15a, 15b) derart angeordnet ist, dass damit die Sendeeinheit (17) und/oder die Empfangseinheit (18) jeweils in einer transversalen Richtung (T) orthogonal zu einer Oberfläche des bandförmigen Materials (1) und/oder in einer longitudinalen Richtung (L) parallel zu einer Oberfläche des bandförmigen Materials (1) beweglich verstellbar sind.

3. Vorrichtung (10) nach Anspruch 1 oder 2, **dadurch gekennzeichnet, dass** das Metallprodukt (1) aus einem bandförmigen Material gebildet ist und außerhalb des Gehäuses (11) eine Halteeinrichtung (H) vorgesehen ist, an der die Sendeeinheit (17) und/oder die Empfangseinheit (18) angebracht sind bzw. ist, wobei die Halteeinrichtung (H) in Bezug auf das Metallprodukt (1) in einer longitudinalen Richtung (L) parallel zu einer Oberfläche des bandförmigen Materials (1) beweglich verstellbar ist.

4. Vorrichtung (10) nach einem der vorhergehenden Ansprüche, **dadurch gekennzeichnet, dass** die Abschirmungen (20) jeweils innerhalb der ersten Öffnung (13) und der zweiten Öffnung (14) befestigt sind und dadurch einen Innenraum (11i) des Gehäuses (11) gegenüber der Umgebung (U) verschließen.

5. Vorrichtung (10) nach einem der Ansprüche 1 bis 3, **dadurch gekennzeichnet, dass** die Abschirmung (20) mit einer insbesondere elastisch verformbaren Dichteinrichtung (23) verbunden ist, wobei die Dichteinrichtung (23) mit den Rändern der ersten bzw. zweiten Öffnung (13, 14) befestigt ist und dadurch der Innenraum (11i) des Gehäuses (11) gegenüber der Umgebung (U) abgedichtet ist,.

6. Vorrichtung (1) nach Anspruch 5, **dadurch gekennzeichnet, dass** die Dichteinrichtung (23) in Form eines verformbaren Faltenbalgs ausgebildet ist, weiter vorzugsweise, dass der Faltenbalg im Querschnitt rund, gerundet, oval, rechteckig, quadratisch oder in einer Kombination dieser Formen ausgebildet ist.

7. Vorrichtung (10) nach Anspruch 5 oder 6, **dadurch gekennzeichnet, dass** die Dichteinrichtung zumindest einen stirnseitigen und/oder seitlichen Abschnitt umfasst, der mit einer Schutzschicht gegen elektromagnetische und/oder thermische Strahlung ausgerüstet ist.

8. Vorrichtung nach einem der Ansprüche 5 bis 7, **gekennzeichnet durch** Verstellmittel (32), mit denen die Dichteinrichtung (23) wirkverbunden ist, wobei bei einer Aktuierung der Verstellmittel (32) die mit der Dichteinrichtung (23) verbundene Abschirmung (20) in das Gehäuse (11) hinein oder aus dem Gehäuse (11) heraus bewegbar ist, vorzugsweise, dass die Verstellmittel (32) teleskopisch ausgebildet sind.

9. Vorrichtung nach einem der Ansprüche 5 bis 8, **dadurch gekennzeichnet, dass** für die Dichteinrichtung (23) eine Längserstreckung umfasst und dass entlang ihrer Längserstreckung Stützmittel (33) vorgesehen sind, die eine Stabilisierung der Dichteinrichtung (23) in ihrer Längsrichtung gewährleisten.

10. Vorrichtung (10) nach einem der vorhergehenden Ansprüche, **dadurch gekennzeichnet, dass** die Verstelleinrichtung (15a, 15b) derart ausgebildet ist, dass eine damit verbundene Komponente (17, 18) der Messvorrichtung (16) in das Gehäuse (11) hinein bewegbar ist, vorzugsweise, dass die Verstelleinrichtung (15a, 15b) teleskopisch ausgebildet ist.

11. Vorrichtung (10) nach einem der vorhergehenden Ansprüche, **gekennzeichnet durch** zumindest eine Kühleinrichtung (24) und/oder zumindest eine Spülgaseinrichtung (F), mit denen die Abschirmungen bzw. Fenster (20) und/oder die Komponenten (17, 18) der Messvorrichtung (16) und/oder die Dichteinrichtung (23) kühlbar und/oder mit einem Spülgas beaufschlagbar sind.

12. Vorrichtung (10) nach Anspruch 11, **dadurch gekennzeichnet, dass** eine Kühleinrichtung (24) zumindest eine Kühlleitung (30) oder eine Kavität umfasst, die in einer Wandung (12) des Gehäuses (11) oder in einem Material der Dichteinrichtung (23) ausgebildet ist, vorzugsweise, dass eine Kühleinrichtung (24) eine Leitungsschlange (25) umfasst, die an einer Komponente (17, 18) der Messvorrichtung (16) angebracht ist, weiter vorzugsweise, dass eine Spülgaseinrichtung (F) angrenzend zu einer Abschirmung (20) und/oder an einer Komponente (17, 18) der Messvorrichtung (16) angebracht ist.

13. Vorrichtung (10) nach einem der vorhergehenden Ansprüche, **dadurch gekennzeichnet, dass** die Abschirmungen bzw. Fenster (20) mehrlagig ausgebildet sind und zwischen den einzelnen Schichten (21) der Abschirmungen bzw. Fenster (20) eine Kavität (K) ausgebildet ist, wobei diese Kavität (K) mit Kühlfluid insbesondere in Form von Kühlgas durchströmt wird.

14. Vorrichtung (10) nach einem der vorhergehenden Ansprüche, **dadurch gekennzeichnet, dass** im Bereich der ersten Öffnung (13) und/oder der zweiten Öffnung (14) jeweils eine Verschlusseinrichtung (26) vorgesehen ist, wobei die erste Öffnung (13) und/oder die zweite Öffnung (14) mittels der Verschlusseinrichtung (26) verschließbar sind.

15. Verfahren zum berührungslosen Ermitteln von zumindest einer Eigenschaft eines Metallprodukts (1) während einer metallurgischen Herstellung des Metallprodukts (1), unter Verwendung einer Vorrichtung (10) nach einem der Ansprüche 1 bis 14.

## Claims

1. Device (10) for the contactless determination of at least one property of a metal product (1) during the metallurgical production of the metal product (1), comprising at least one measuring device (16) consisting of a transmitting unit (17) and a receiving unit (18),
wherein an electromagnetic field is generated at least by the transmitting unit (17) and directed onto the metal product (1) and thereby a physical interaction is induced in the material of the metal product (1),
wherein a remaining and/or resulting part of this physical interaction is received by the receiving unit (18),
**characterized in that** the device (10) comprises a housing (11) through which the metal product (1) is movable, the housing (11) being part of a furnace for heat treatment and/or part of a plant for coating the metal product (1);
wherein a first opening (13) and a second opening (14) are formed in the wall (12) of the housing (11), wherein the transmitting unit (17) is associated with the first opening (13), such that the electromagnetic field generated by the transmitting unit (17) impinges on the metal product (1) on the side of the first opening (13), wherein the receiving unit (18) is associated with the second opening (14), such that the remaining and/or resulting part of the physical interaction induced in the material of the metal product (1) is received by the receiving unit (18) on the side of the second opening (14),
wherein the housing (11) has a narrow point (19) in the region of the first opening (13) and/or the second opening (14),
wherein in the region of the first opening (13) and of the second opening (14), there is provided in each case a shield (20), in the form of a window, which is transparent with respect to the waves of electromagnetic radiation or an associated physical effect,
wherein the shield (20) is designed in such a manner to reduce heat radiation through the openings (13, 14) from the interior space (11i) of the housing (11) to its surrounding area (U) where the transmitting unit (17) and the receiving unit (18) are located; and
wherein, at least one adjusting device (15a, 15b) is provided outside the housing (11), by means of which at least one component (17, 18) of the measuring device (16) consisting of the transmitting unit (17) and/or the receiving unit (18) can be moved relative to the wall (12) of the housing (11) and in the region of an opening (13, 14) of the housing (11) or adjacent thereto, in order to thereby set or selectively change for such component (17, 18) a predetermined distance to the metal product (1) moved within the housing (11).

2. Device (10) according to claim 1, **characterized in that** the metal product (1) is formed of a strip-shaped material, wherein the adjusting device (15a, 15b) is arranged in such a manner that, thereby, the transmitting unit (17) and/or the receiving unit (18) are each movably adjustable in a transverse direction (T) orthogonal to a surface of the strip-shaped material (1) and/or in a longitudinal direction (L) parallel to a surface of the strip-shaped material (1).

3. Device (10) according to claim 1 or 2, **characterized in that** the metal product (1) is formed of a strip-shaped material and a holding device (H) is provided outside the housing (11), to which the transmitting unit (17) and/or the receiving unit (18) is/are attached, wherein the holding device (H) is movably adjustable with respect to the metal product (1) in a longitudinal direction (L) parallel to a surface of the strip-shaped material (1).

4. Device (10) according to one of the preceding claims, **characterized in that** the shields (20) are respectively fastened inside the first opening (13) and the second opening (14), thereby closing an interior space (11i) of the housing (11) with respect to the surrounding area (U).

5. Device (10) according to any of claims 1 to 3, **characterized in that** the shield (20) is connected to a sealing device (23), in particular an elastically deformable sealing device (23), wherein the sealing device (23) is fastened to the edges of the first and second openings (13, 14), respectively, and thereby sealing the interior space (11i) of the housing (11) with respect to the surrounding area (U).

6. Device (10) according to claim 5, **characterized in that** the sealing device (23) is in the form of a deformable bellows, more preferably **in that** the bellows is round, rounded, oval, rectangular, square in cross-section or a combination of such shapes.

7. Device (10) according to claim 5 or 6, **characterized in that** the sealing device (23) comprises at least a frontal and/or lateral section equipped with a protective layer against electromagnetic and/or thermal radiation.

8. Device according to one of the claims 5 to 7, **characterized by** adjusting means (32) to which the sealing device (23) is operatively connected, wherein, upon actuation of the adjusting means (32) the shield (20) connected to the sealing device (23) is movable into or out of the housing (11), preferably in that the adjusting means (32) is formed to be telescopic.

9. Device according to one of the claims 5 to 8, **characterized in that** the sealing device (23) comprises a longitudinal extension, and **in that** support means (33) is provided for the sealing device (23) along its longitudinal extension, which support means (33) ensures stabilization of the sealing device (23) in its longitudinal direction.

10. Device (10) according to one of the preceding claims, **characterized in that** the adjusting device (15a, 15b) is designed in such a manner that a component (17, 18) of the measuring device (16) connected thereto is movable into the housing (11), preferably **in that** the adjusting device (15a, 15b) is formed to be telescopic.

11. Device (10) according to one of the preceding claims, **characterized by** at least one cooling device (24) and/or at least one purge gas device (F), by means of which the shields or windows (20), as the case may be, and/or the components (17, 18) of the measuring device (16) and/or the sealing device (23) can be cooled and/or can be acted upon by a purge gas.

12. Device (10) according to claim 11, **characterized in that** a cooling device (24) comprises at least one cooling line (30) or cavity that is formed in a wall (12) of the housing (11) or in a material of the sealing device (23), preferably **in that** a cooling device (24) comprises a line coil (25) that is attached to a component (17, 18) of the measuring device (16), further preferably that a purge gas device (F) is attached in a manner adjacent to a shield (20) and/or to a component (17, 18) of the measuring device (16).

13. Device (10) according to one of the preceding claims, **characterized in that** the shields or windows (20), as the case may be, are formed in multiple layers and a cavity (K) is formed between the individual layers (21) of the shields or windows (20), as the case may be, wherein such cavity (K) is flowed through with cooling fluid, in particular in the form of cooling gas.

14. Device (10) according to one of the preceding claims, **characterized in that** a closing device (26) is provided in each case in the region of the first opening (13) and/or of the second opening (14), wherein the first opening (13) and/or the second opening (14) is closable by means of the closing device (26).

15. Method for the contactless determination of at least one property of a metal product (1) during the metallurgical production of the metal product (1), the method using a device (10) according to one of the claims 1 to 14.

## Revendications

1. Dispositif (10) pour la détermination sans contact d'au moins une propriété d'un produit métallique (1) au cours de la fabrication métallurgique dudit produit métallique (1), comprenant au moins un dispositif de mesure (16) constitué d'une unité d'émission (17) et d'une unité de réception (18),
dans lequel au moins l'unité d'émission (17) génère un champ électromagnétique qui est dirigé vers le produit métallique (1) et induit ainsi une interaction physique dans le matériau du produit métallique (1), une partie résiduelle et/ou résultante de cette interaction physique étant captée par l'unité de réception (18),
**caractérisé en ce que** le dispositif (10) comprend une enceinte (11) à travers lequel le produit métallique (1) peut être déplacé, l'enceinte (11) faisant partie d'un four de traitement thermique et/ou d'une installation de revêtement du produit métallique (1);
dans lequel une première ouverture (13) et une deuxième ouverture (14) sont formées dans la paroi (12) de l'enceinte (11), l'unité d'émission (17) étant associée à la première ouverture (13), de sorte que le champ électromagnétique généré par l'unité d'émission (17) frappe le produit métallique (1) du côté de la première ouverture (13), l'unité de réception (18) étant associée à la deuxième ouverture (14), de sorte que la partie résiduelle et/ou résultante de l'interaction physique induite dans le matériau du produit métallique (1) est reçue par l'unité de réception (18) du côté de la deuxième ouverture (14),
dans lequel l'enceinte (11) présente, dans la zone de la première ouverture (13) et/ou de la deuxième ouverture (14), un rétrécissement (19),
dans lequel un écran (20), notamment sous la forme d'une fenêtre, est prévu dans la zone de chacune de la première ouverture (13) et de la deuxième ouverture (14), lequel est perméable aux ondes du rayonnement électromagnétique ou à un effet physique associé,
dans lequel l'écran (20) est conçu de telle sorte qu'il réduit le rayonnement thermique traversant les ouvertures (13, 14) depuis l'espace intérieur (11i) de l'enceinte (11) vers son environnement (U) dans lequel se trouvent l'unité d'émission (17) et l'unité de réception (18); et
dans lequel au moins un dispositif de réglage (15a, 15b) est prévu à l'extérieur de l'enceinte (11), grâce auquel au moins un composant (17, 18) du dispositif de mesure (16) constitué de l'unité d'émission (17) et/ou de l'unité de réception (18) peut être déplacé par rapport à la paroi (12) de l'enceinte (11) et dans la zone d'une ouverture (13, 14) de l'enceinte (11) ou à proximité de celle-ci, afin de régler ou de modifier sélectivement, pour ce composant (17, 18), une distance prédéterminée par rapport au produit métallique (1) se déplaçant à l'intérieur de l'enceinte (11).

2. Dispositif (10) selon la revendication 1, **caractérisé en ce que** le produit métallique (1) est constitué d'un matériau en forme de bande, le dispositif de réglage (15a, 15b) étant agencé de sorte que l'unité d'émission (17) et/ou l'unité de réception (18) puissent être déplacées respectivement dans une direction transversale (T) orthogonale à une surface du matériau en forme de bande (1) et/ou dans une direction longitudinale (L) parallèle à une surface du matériau en forme de bande (1).

3. Dispositif (10) selon la revendication 1 ou 2, **caractérisé en ce que** le produit métallique (1) est formé d'un matériau en forme de bande et qu'un dispositif de maintien (H) est prévu à l'extérieur de l'enceinte (11), sur lequel sont montés l'unité d'émission (17) et/ou l'unité de réception (18), le dispositif de maintien (H) étant réglable de manière mobile par rapport au produit métallique (1) dans une direction longitudinale (L) parallèle à une surface du matériau en forme de bande (1).

4. Dispositif (10) selon l'une des revendications précédentes, **caractérisé en ce que** les écrans (20) sont fixés respectivement à l'intérieur de la première ouverture (13) et de la deuxième ouverture (14) et ferment ainsi un espace intérieur (11i) de l'enceinte (11) par rapport à l'environnement (U).

5. Dispositif (10) selon l'une des revendications 1 à 3, **caractérisé en ce que** l'écran (20) est relié à un dispositif d'étanchéité (23) notamment déformable élastiquement, le dispositif d'étanchéité (23) étant fixé aux bords des première et deuxième ouvertures (13, 14), ce qui permet de rendre étanche l'espace intérieur (11i) de l'enceinte (11) par rapport à l'environnement (U).

6. Dispositif (10) selon la revendication 5, **caractérisé en ce que** le dispositif d'étanchéité (23) se présente sous la forme d'un soufflet déformable, et de préférence **en ce que** le soufflet présente, en section transversale, une forme ronde, arrondie, ovale, rectangulaire, carrée ou une combinaison de ces formes.

7. Dispositif (10) selon la revendication 5 ou 6, **caractérisé en ce que** le dispositif d'étanchéité comprend au moins une partie frontale et/ou latérale équipée d'une couche de protection contre le rayonnement électromagnétique et/ou thermique.

8. Dispositif selon l'une des revendications 5 à 7, **caractérisé par** des moyens de réglage (32) auxquels le dispositif d'étanchéité (23) est relié de manière fonctionnelle, dans lequel, lors de l'actionnement des moyens de réglage (32), l'écran (20) relié au dispositif d'étanchéité (23) peut être déplacé vers l'intérieur ou hors de l'enceinte (11), de préférence en ce que les moyens de réglage (32) sont de type télescopique.

9. Dispositif selon l'une des revendications 5 à 8, **caractérisé en ce que** le dispositif d'étanchéité (23) présente une extension longitudinale et **en ce que** des moyens de support (33) sont prévus le long de cette extension longitudinale, lesquels assurent la stabilisation du dispositif d'étanchéité (23) dans sa direction longitudinale.

10. Dispositif (10) selon l'une des revendications précédentes, **caractérisé en ce que** le dispositif de réglage (15a, 15b) est conçu de telle sorte qu'un composant (17, 18) du dispositif de mesure (16) qui lui est relié est déplaçable à l'intérieur de l'enceinte (11), de préférence **en ce que** le dispositif de réglage (15a, 15b) est de type télescopique.

11. Dispositif (10) selon l'une des revendications précédentes, **caractérisé par** au moins un dispositif de refroidissement (24) et/ou au moins un dispositif de gaz de purge (F), grâce auxquels les écrans ou fenêtres (20), et/ou les composants (17, 18) du dispositif de mesure (16) et/ou le dispositif d'étanchéité (23) peuvent être refroidis et/ou alimentés en gaz de purge.

12. Dispositif (10) selon la revendication 11, **caractérisé en ce qu'**un dispositif de refroidissement (24) comprend au moins une conduite de refroidissement (30) ou cavité de refroidissement formée dans une paroi (12) de l'enceinte (11) ou dans un matériau du dispositif d'étanchéité (23), de préférence **en ce qu'**un dispositif de refroidissement (24) comprend un serpentin (25) qui est monté sur un composant (17, 18) du dispositif de mesure (16), et plus préférablement **en ce qu'**un dispositif de gaz de purge (F) est monté à proximité d'un écran (20) et/ou sur un composant (17, 18) du dispositif de mesure (16).

13. Dispositif (10) selon l'une des revendications précédentes, **caractérisé en ce que** les écrans ou fenêtres (20) sont constitués de plusieurs couches et qu'une cavité (K) est formée entre les différentes couches (21) des écrans ou fenêtres (20), respectivement, cette cavité (K) étant traversée par un fluide de refroidissement, notamment sous forme de gaz de refroidissement.

14. Dispositif (10) selon l'une des revendications précédentes, **caractérisé en ce qu'**un dispositif de fermeture (26) est prévu respectivement dans la zone de la première ouverture (13) et/ou de la deuxième ouverture (14), la première ouverture (13) et/ou la deuxième ouverture (14) pouvant être fermées au moyen du dispositif de fermeture (26).

15. Procédé de détermination sans contact d'au moins une propriété d'un produit métallique (1) au cours de la fabrication métallurgique dudit produit métallique (1), le procédé utilisant un dispositif (10) selon l'une des revendications 1 à 14.
